(19) Europäisches Patentamt / European Patent Office / Office européen des brevets

(11) **EP 1 331 484 B1**

(12) **EUROPEAN PATENT SPECIFICATION**

(45) Date of publication and mention
of the grant of the patent:
**14.11.2007 Bulletin 2007/46**

(51) Int Cl.:
*G01N 33/58* (2006.01)     *G01N 21/76* (2006.01)
*G06F 19/00* (2006.01)

(21) Application number: **03001295.9**

(22) Date of filing: **22.01.2003**

(54) **Chemiluminescence method for producing biochemical analysis data and apparatus used therefor**

Chemilumineszenzverfahren zur Erzeugung von daten der biochemischen Analyse und Vorrichtung dazu

Procédé de chimioluminescence pour la production de données d'analyse biochimique et dispositif adapté

(84) Designated Contracting States:
**DE FR GB SE**

(30) Priority: **29.01.2002 JP 2002019840**

(43) Date of publication of application:
**30.07.2003 Bulletin 2003/31**

(73) Proprietor: **FUJIFILM Corporation
Minato-ku
Tokyo (JP)**

(72) Inventor: **Ogura, Nobuhiko
Kaisei-machi,
Ashigarakami-gun,
Kanagawa (JP)**

(74) Representative: **Klunker . Schmitt-Nilson . Hirsch
Winzererstrasse 106
80797 München (DE)**

(56) References cited:
**EP-A- 0 824 211          WO-A-00/40334
WO-A-01/67369          GB-A- 2 315 130
US-A- 4 922 092          US-A- 5 422 075
US-B1- 6 171 793**

- **PELIZZARI C. A. ET AL: "Quantitative analysis of DNA array autoradiographs" NUCLEIC ACID RESEARCH, vol. 28, no. 22, 2000, pages 4577-4581,**
- **VOHRADSKY J. ET AL: "Developmental control of stress stimulons in Streptomyces coelicolor revealed by statistical analysis of global gene expression patterns" JOURNAL OF BACTERIOLOGY, vol. 182, no. 17, September 2000 (2000-09), pages 4979-4986,**

## Description

## BACKGROUND OF THE INVENTION

The present invention relates to a method for producing biochemical analysis data and an apparatus used therefor and, particularly, to a method for producing biochemical analysis data and an apparatus used therefor which can in a desired manner detect chemiluminescence emission released from a specimen region containing a small amount of a labeling substance which generates chemiluminescence emission when it contacts a chemiluminescent substrate as well as chemiluminescence emission released from a specimen region containing a large amount of the labeling substance, thereby producing biochemical analysis data having an excellent quantitative characteristic.

## DESCRIPTION OF THE PRIOR ART

An autoradiographic analyzing system using as a detecting material for detecting radiation a stimulable phosphor which can absorb, store and record the energy of radiation when it is irradiated with radiation and which, when it is then stimulated by an electromagnetic wave having a specified wavelength, can release stimulated emission whose light amount corresponds to the amount of radiation with which it was irradiated is known, which comprises the steps of introducing a radioactively labeled substance into an organism, using the organism or a part of the tissue of the organism as a specimen, superposing the specimen and a stimulable phosphor sheet formed with a stimulable phosphor layer for a certain period of time, storing and recording radiation energy in a stimulable phosphor contained in the stimulable phosphor layer, scanning the stimulable phosphor layer with an electromagnetic wave to excite the stimulable phosphor, photoelectrically detecting the stimulated emission released from the stimulable phosphor to produce digital image signals, effecting image processing on the obtained digital image signals, and reproducing an image on displaying means such as a CRT or the like or a photographic film (see, for example, Japanese Patent Publication No. 1-60784, Japanese Patent Publication No. 1-60782, Japanese Patent Publication No. 4-3952 and the like).

Unlike the system using a photographic film, according to the autoradiographic analyzing system using the stimulable phosphor as a detecting material for detecting radiation, development, which is chemical processing, becomes unnecessary. Further, it is possible reproduce a desired image by effecting image processing on the obtained image data and effect quantitative analysis using a computer. Use of a stimulable phosphor in these processes is therefore advantageous.

On the other hand, a fluorescence analyzing system using a fluorescent substance as a labeling substance instead of a radioactive labeling substance in the autoradiographic analyzing system is known. According to this system, it is possible to study a genetic sequence, study the expression level of a gene, and to effect separation or identification of protein or estimation of the molecular weight or properties of protein or the like. For example, this system can perform a process including the steps of distributing a plurality of DNA fragments on a gel support by means of electrophoresis after a fluorescent dye was added to a solution containing a plurality of DNA fragments to be distributed, or distributing a plurality of DNA fragments on a gel support containing a fluorescent dye, or dipping a gel support on which a plurality of DNA fragments have been distributed by means of electrophoresis in a solution containing a fluorescent dye, thereby labeling the electrophoresed DNA fragments, exciting the fluorescent dye by a stimulating ray to cause it to release fluorescence emission, detecting the released fluorescence emission to produce an image and detecting the distribution of the DNA fragments on the gel support. This system can also perform a process including the steps of distributing a plurality of DNA fragments on a gel support by means of electrophoresis, denaturing the DNA fragments, transferring at least a part of the denatured DNA fragments onto a transfer support such as a nitrocellulose support by the Southern-blotting method, hybridizing a probe prepared by labeling target DNA and DNA or RNA complementary thereto with the denatured DNA fragments, thereby selectively labeling only the DNA fragments complementary to the probe DNA or probe RNA, exciting the fluorescent dye by a stimulating ray to cause it to release fluorescence emission, detecting the released fluorescence emission to produce an image and detecting the distribution of the target DNA on the transfer support. This system can further perform a process including the steps of preparing a DNA probe complementary to DNA containing a target gene labeled by a labeling substance, hybridizing it with DNA on a transfer support, combining an enzyme with the complementary DNA labeled by a labeling substance, causing the enzyme to contact a fluorescent substance, transforming the fluorescent substance to a fluorescent substance having fluorescence emission releasing property, exciting the thus produced fluorescent substance by a stimulating ray to release fluorescence emission, detecting the fluorescence emission to produce an image and detecting the distribution of the target DNA on the transfer support. This fluorescence detecting system is advantageous in that a genetic sequence or the like can be easily detected without using a radioactive substance.

Similarly, there is known a chemiluminescence detecting system comprising the steps of fixing a substance derived from a living organism such as a protein or a nucleic acid sequence on a support, selectively labeling the substance derived from a living organism with a labeling substance which generates chemiluminescence emission when it contacts a chemiluminescent substrate, contacting the substance derived from a living organism and selectively labeled with the labeling substance and the chemiluminescent substrate, photoelectrically detecting the chemiluminescence emission in the wavelength of visible light generated by the contact of the chemiluminescent substrate and the labeling substance to produce digital image signals, effecting image processing thereon, and reproducing a chemiluminescent image on a display means such as a CRT or a recording material such as a photographic film, thereby obtaining information relating to the high molecular substance such as genetic information.

A CCD camera is normally used for photoelectrically detecting chemiluminescence emission and producing biochemical analysis data.

However, since the dynamic range of a CCD camera is normally a on the order of three digits, if the exposure time of the CCD camera to chemiluminescence emission is set longer in order to detect chemiluminescence emission having a low intensity and released from a specimen region containing a small amount of a labeling substance which generates chemiluminescence emission when it contacts a chemiluminescent substrate and produce biochemical analysis data having an excellent quantitative characteristic, it is impossible to produce biochemical analysis data having an excellent quantitative characteristic by detecting chemiluminescence emission released from a specimen region containing a large amount of a labeling substance which generates chemiluminescence emission when it contacts a chemiluminescent substrate, since the intensity of the chemiluminescence emission is extremely high and the number of photons generated by the chemiluminescence emission and entering the photo-electric detecting surface of the CCD camera exceeds the upper limit of the dynamic range of the CCD camera. On the other hand, if the exposure time of the CCD camera to chemiluminescence emission is set shorter in order to detect chemiluminescence emission released from a specimen region containing a large amount of a labeling substance which generates chemiluminescence emission when it contacts a chemiluminescent substrate and produce biochemical analysis data having an excellent quantitative characteristic, the quantitative characteristic of biochemical analysis data produced by detecting chemiluminescence emission having a low intensity and released from a specimen region containing a small amount of the labeling substance are extremely lowered and biochemical analysis data having an excellent quantitative characteristic cannot be obtained.

US 5 422 075 discloses a system which makes use of two different optical detectors having high sensitivity and low sensitivity.

SUMMARY OF THE INVENTION

It is therefore an object of the present invention to provide a method for producing biochemical analysis data and an apparatus used therefor which can in a desired manner detect chemiluminescence emission released from a specimen region containing a small amount of a labeling substance which generates chemiluminescence emission when it contacts a chemiluminescent substrate as well as chemiluminescence emission released from a specimen region containing a large amount of the labeling substance, thereby producing biochemical analysis data having an excellent quantitative characteristic.

The above and other objects of the present invention can be accomplished by a method for producing biochemical analysis data comprising the steps of claim 1.

In the case where biochemical analysis data are produced by photoelectrically detecting chemiluminescence emission, a solid state area sensor such as a CCD area sensor is generally used. However, if the exposure time of the solid state area sensor to chemiluminescence emission is set long in order to detect chemiluminescence emission released from an absorptive region of the biochemical analysis unit containing a small amount of a labeling substance which generates chemiluminescence emission when it contacts a chemiluminescent substrate and having a low intensity and to produce biochemical analysis data having an excellent quantitative characteristic, it then becomes impossible to produce biochemical analysis data having an excellent quantitative characteristic by detecting chemiluminescence emission released from an absorptive region of the biochemical analysis unit containing a large amount of a labeling substance which generates chemiluminescence emission when it contacts a chemiluminescent substrate. This is because in the latter case the intensity of the chemiluminescence emission is extremely high and the number of photons generated by the chemiluminescence emission and entering the photo-electric detecting surface of the solid state area sensor exceeds the upper limit of the dynamic range of the solid state area sensor. On the other hand, if the exposure time of the solid state area sensor to chemiluminescence emission is set short in order to detect chemiluminescence emission released from an absorptive region of the biochemical analysis unit containing a large amount of a labeling substance which generates chemiluminescence emission when it contacts a chemiluminescent substrate and to produce biochemical analysis data having an excellent quantitative characteristic, the quantitative characteristic of biochemical analysis data produced by detecting chemiluminescence emission released from an absorptive region of the biochemical analysis unit containing a small amount of the labeling substance and having a low intensity are extremely lowered and biochemical analysis data having an excellent quantitative characteristic cannot be obtained. To the contrary, according to the present invention, a method for producing biochemical analysis data is provided that comprises the steps of selectively binding a substance derived from a living organism and labeled with a labeling substance which generates chemiluminescence emission when it contacts a chemiluminescent substrate with specific binding substances whose structure and characteristics are known and which are contained in a plurality of absorptive regions formed in a biochemical analysis unit so as to be spaced apart from each other, or selectively binding a substance derived from a living organism and labeled with a hapten with the specific binding substances whose structure and characteristics are known and which are contained in the plurality of absorptive regions formed in the biochemical analysis unit so as to be spaced apart from each other and binding an antibody for the hapten labeled with an enzyme having a property to generate chemiluminescence emission when it contacts a chemiluminescent substrate with the hapten by an antigen-antibody reaction, bringing the labeling substance selectively contained in the plurality of absorptive regions of the biochemical analysis unit into contact with a chemiluminescent substrate, thereby causing it to release chemiluminescence emission, photoelectrically detecting the chemiluminescence emission released from the plurality of absorptive regions of the biochemical analysis unit for a first exposure time period using a solid state area sensor to produce an analog signal for each of the plurality of absorptive regions of the biochemical analysis unit, digitizing the analog signal to produce a digital signal for each of the plurality of absorptive regions of the biochemical analysis unit, storing the digital signals in a memory, photoelectrically detecting the chemiluminescence emission released from the plurality of absorptive regions of the biochemical analysis unit for a second exposure time period longer than the first exposure time period to produce an analog signal for each of the plurality of absorptive regions of the biochemical analysis unit, digitizing the analog signal to produce a digital signal for each of the plurality of absorptive regions of the biochemical analysis unit, storing the digital signals in the memory, comparing a signal intensity of the digital signal produced by photoelectrically detecting the chemiluminescence emission released from each of the absorptive regions of the biochemical analysis unit for the second exposure time period and stored in the memory with a saturated value of the signal intensity, determining a signal intensity of a digital signal which is lower than the saturated value as biochemical analysis data of the corresponding absorptive region of the biochemical analysis unit to store it in the memory, and adopting a signal intensity of a digital signal which is generated by photoelectrically detecting the chemiluminescence emission released from an absorptive region of the biochemical analysis unit for the first exposure time period and stored in the memory when a signal intensity of a digital signal generated by photoelectrically detecting the chemiluminescence emission released from the absorptive region of the biochemical analysis unit for the second exposure time period is equal to or higher than the saturated value, thereby producing biochemical analysis data. Therefore, while the conventional method is incapable of producing biochemical analysis data having an excellent quantitative characteristic by using a solid state area sensor to detect chemiluminescence emission released from an absorptive region containing an extremely large amount of a labeling substance which generates chemiluminescence emission when it contacts a chemiluminescent substrate because the intensity of the chemiluminescence emission is extremely high and the number of photons generated by the chemiluminescence emission and entering the photo-electric detecting surface of the solid state area sensor exceeds the dynamic range of the solid state area sensor, the method of the present invention can produce biochemical analysis data having an excellent quantitative characteristic even in such a case. On the other hand, while the conventional method cannot easily produce biochemical analysis data having an excellent quantitative characteristic by using a solid state area sensor to photoelectrically detect chemiluminescence emission because the absorptive region contains only a small amount of the labeling substance which generates chemiluminescence emission when it contacts a chemiluminescent substrate and the intensity of chemiluminescence emission released from the absorptive region is low, the method of the present invention can produce biochemical analysis data having an excellent quantitative characteristic even in such a case because the signal intensity of a digital signal obtained by photoelectrically detecting chemiluminescence emission released from the absorptive region of the biochemical analysis unit for the second exposure time period considerably longer than the first exposure time period is adopted as biochemical analysis data of the absorptive region.

In a preferred aspect of the present invention, the method of producing biochemical analysis data further comprises the steps of producing a correction coefficient as defined by claim 2.

According to this preferred aspect of the present invention, the method of producing biochemical analysis data further includes the steps of producing a correction coefficient based on a ratio of a signal intensity of a digital signal generated by photoelectrically detecting chemiluminescence emission released from an absorptive region of the biochemical analysis unit for the second exposure time period and stored in the memory and whose signal intensity is lower than the saturated value and a signal intensity of a digital signal generated by photoelectrically detecting chemiluminescence emission released from the absorptive region of the biochemical analysis unit for the first exposure time period and stored in the memory, and multiplying a signal intensity of the digital signal generated by photoelectrically detecting chemiluminescence emission released from an absorptive region of the biochemical analysis unit for the second exposure time period and whose signal intensity is equal to or higher than the saturated value by the correction coefficient, thereby producing biochemical analysis data of the absorptive region. Hence, the signal intensity of the digital signal generated by photoelectrically detecting chemiluminescence emission released from an absorptive region of the biochemical analysis unit for the first exposure time period, stored in the memory and adopted as biochemical analysis data of the absorptive region of the biochemical analysis unit when the signal intensity of the digital signal generated by photoelectrically detecting chemiluminescence emission released from the absorptive region of the biochemical analysis unit for the second exposure time period and stored in the memory is equal to or higher than the saturated value is corrected so as to be equivalent to that generated by photoelectrically detecting chemiluminescence emission released from an absorptive region of the biochemical analysis unit for the second exposure time period similarly to the signal intensity of the digital signal generated by photoelectrically detecting chemiluminescence emission released from an absorptive region of the biochemical analysis unit for the second exposure time period and stored in the memory and whose signal intensity is lower than the saturated value. Therefore, quantitative analysis can be effected with high accuracy in a desired manner by comparing the signal intensities of the digital signals generated by photoelectrically detecting chemiluminescence emission released from the plurality of absorptive regions of the biochemical analysis unit.

In a preferred aspect of the present invention, the method for producing biochemical analysis data further comprises the step of dividing a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient.

According to this preferred aspect of the present invention, since the method for producing biochemical analysis data further comprises the step of dividing a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient, it is possible to produce biochemical analysis data having a more excellent quantitative characteristic.

In a further preferred aspect of the present invention, biochemical analysis data are produced by leading chemiluminescence emission released from the plurality of absorptive regions of the biochemical analysis unit by a plurality of light guide members disposed in such a manner that each of the plurality of absorptive regions faces one of the light collecting end portions of the plurality of light guide members to a light detector and photoelectrically detecting chemiluminescence emission by the light detector.

According to this preferred aspect of the present invention, even in the case of two-dimensionally forming a plurality of absorptive regions in a substrate of a biochemical analysis unit with high density so as to be spaced apart from each other, spotting specific binding substances which can specifically bind with a substance derived from a living organism and whose sequence, base length, composition and the like are known into the plurality of absorptive regions, specifically binding the specific binding substances contained in the plurality of absorptive regions with a substance derived from a living organism and labeled with a labeling substance which generates chemiluminescence emission when it contacts a chemiluminescent substrate by means of hybridization or the like, thereby selectively labeling the plurality of absorptive regions, when the biochemical analysis unit is placed on a sample stage and chemiluminescence emission released from the plurality of absorptive regions of the biochemical analysis unit is photoelectrically detected to produce biochemical analysis data, chemiluminescence emission released from the plurality of absorptive regions can be led to a solid state area sensor with high light collecting efficiency and photoelectrically detected by the solid state area sensor by disposing the plurality of light guide members in such a manner that each of the plurality of light guide members is sufficiently close to one of the plurality of absorptive regions two-dimensionally formed in the biochemical analysis unit so as to be spaced apart from each other, receiving chemiluminescence emission released from each of the absorptive regions by one of the light collecting end portions of the plurality of light guide members to lead it to the solid state area sensor and photoelectrically detecting chemiluminescence emission by the solid state area sensor. Therefore, it is possible to produce biochemical analysis data having excellent quantitative characteristics with high resolution.

In a preferred aspect of the present invention, each of the plurality of light guide members is formed of at least one optical fiber.

In another preferred aspect of the present invention, each of the plurality of light guide members is formed of an optical fiber bundle constituted by a plurality of optical fibers.

In a preferred aspect of the present invention, the plurality of light guide members are gathered in the vicinity of end portions opposite to the light collecting end portions.

According to this preferred aspect of the present invention, since the plurality of light guide members are gathered in the vicinity of end portions opposite to the light collecting end portions, it is possible to employ a two-dimensional sensor having a small light detecting surface, thereby enabling an apparatus for producing biochemical analysis data to be smaller and lowering cost for manufacturing it.

In a preferred aspect of the present invention, the plurality of light guide members are mounted on a fixing head in the vicinity of the light collecting end portions so that each of the light collecting end portions of the plurality of light guide members is disposed to face one of the plurality of absorptive regions.

In a further preferred aspect of the present invention, the solid state area sensor is constituted by a cooled CCD area sensor.

According to this preferred aspect of the present invention, since the solid state area sensor is constituted by a cooled CCD area sensor, it is possible to collect weak chemiluminescence emission released from the plurality of absorptive regions of the biochemical analysis unit by the plurality of light guide members and photoelectrically detect it for a long time and, therefore, it is possible to detect chemiluminescence emission with sufficiently high sensitivity to produce biochemical analysis data.

The above and other objects of the present invention can be also accomplished by an apparatus for producing biochemical analysis data comprising a sample stage for placing a biochemical analysis unit in which a plurality of absorptive regions are formed so as to be spaced apart from each other and a substance derived from a living organism and labeled with a labeling substance which generates chemiluminescence emission when it contacts a chemiluminescent substrate is selectively bound with specific binding substances whose structure or characteristics are known contained in the plurality of absorptive regions, a solid state area sensor for photoelectrically detecting chemiluminescence emission released from the plurality of absorptive regions of the biochemical analysis unit and generating an analog signal for each of the plurality of absorptive regions of the biochemical analysis unit, a plurality of light guide members disposed in such a manner that each of light collecting end portions of the light guide members faces one of the plurality of absorptive regions of the biochemical analysis unit placed on the sample stage and adapted for leading chemiluminescence emission released from the plurality of absorptive regions of the biochemical analysis unit to the solid state area sensor, an A/D converter for digitizing analog signals generated by the solid state area sensor to produce a digital signal for each of the plurality of absorptive regions of the biochemical analysis unit, a memory for storing the digital signal generated by the A/D converter for each of the plurality of absorptive regions of the biochemical analysis unit, data processing means for producing biochemical analysis data for each of the plurality of absorptive regions of the biochemical analysis unit based on the digital signal of each of the plurality of absorptive regions of the biochemical analysis unit, and a control means for controlling the solid state area sensor, the A/D converter and the data processing means, the control means being adapted for controlling the solid state area sensor so as to photoelectrically detect chemiluminescence emission released from the absorptive regions of the biochemical analysis unit for a first exposure time period, thereby generating an analog signal for each of the absorptive regions of the biochemical analysis unit and to photoelectrically detect chemiluminescence emission released from the absorptive regions of the biochemical analysis unit for a second exposure time period longer than the first exposure time period, thereby generating an analog signal for each of the absorptive regions of the biochemical analysis unit, controlling the A/D converter so as to digitize the analog signal of each of the absorptive regions of the biochemical analysis unit generated by the solid state area sensor and to store it in the memory, and controlling the data processing means to compare a signal intensity of the digital signal of each of the absorptive regions of the biochemical analysis unit generated by photoelectrically detecting chemiluminescence emission released from the absorptive regions of the biochemical analysis unit for the second exposure time period and stored in the memory with a saturated value of the signal intensity, to determine the signal intensity of the digital signal which is lower than the saturated value as biochemical analysis data of the absorptive region of the biochemical analysis unit, to store them in the memory, and to adopt a signal intensity of a digital signal which is generated by photoelectrically detecting the chemiluminescence emission released from an absorptive region of the biochemical analysis unit for the first exposure time period and stored in the memory when a signal intensity of a digital signal generated by photoelectrically detecting the chemiluminescence emission released from the absorptive region of the biochemical analysis unit for the second exposure time period is equal to or higher than the saturated value, thereby producing biochemical analysis data of the plurality of the biochemical analysis unit.

According to the present invention, the apparatus for producing biochemical analysis data comprises a sample stage for placing a biochemical analysis unit in which a plurality of absorptive regions are formed so as to be spaced apart from each other and a substance derived from a living organism and labeled with a labeling substance which generates chemiluminescence emission when it contacts a chemiluminescent substrate is selectively bound with specific binding substances whose structure or characteristics are known contained in the plurality of absorptive regions, a solid state area sensor for photoelectrically detecting chemiluminescence emission released from the plurality of absorptive regions of the biochemical analysis unit and generating an analog signal for each of the plurality of absorptive regions of the biochemical analysis unit, a plurality of light guide members disposed in such a manner that each of light collecting end portions of the light guide members faces one of the plurality of absorptive regions of the biochemical analysis unit placed on the sample stage and adapted for leading chemiluminescence emission released from the plurality of absorptive regions of the biochemical analysis unit to the solid state area sensor, an A/D converter for digitizing analog signals generated by the solid state area sensor to produce a digital signal for each of the plurality of absorptive regions of the biochemical analysis unit, a memory for storing the digital signal generated by the A/D converter for each of the plurality of absorptive regions of the biochemical analysis unit, data processing means for producing biochemical analysis data for each of the plurality of absorptive regions of the biochemical analysis unit based on the digital signal of each of the plurality of absorptive regions of the biochemical analysis unit, and a control means for controlling the solid state area sensor, the A/D converter and the data processing means, the control means is adapted for controlling the solid state area sensor so as to photoelectrically detect chemiluminescence emission released from the absorptive regions of the biochemical analysis unit for a first exposure time period, thereby generating an analog signal for each of the absorptive regions of the biochemical analysis unit and to photoelectrically detect chemiluminescence emission released from the absorptive regions of the biochemical analysis unit for a second exposure time period longer than the first exposure time period, thereby generating an analog signal for each of the absorptive regions of the biochemical analysis unit, controlling the A/D converter so as to digitize the analog signal of each of the absorptive regions of the biochemical analysis unit generated by the solid state area sensor and to store it in the memory, and controlling the data processing means to compare a signal intensity of the digital signal of each of the absorptive regions of the biochemical analysis unit generated by photoelectrically detecting chemiluminescence emission released from the absorptive regions of the biochemical analysis unit for the second exposure time period and stored in the memory with a saturated value of the signal intensity, to determine the signal intensity of the digital signal which is lower than the saturated value as biochemical analysis data of the absorptive region of the biochemical analysis unit, to store them in the memory, and to adopt a signal intensity of a digital signal which is generated by photoelectrically detecting the chemiluminescence emission released from an absorptive region of the biochemical analysis unit for the first exposure time period and stored in the memory when a signal intensity of a digital signal generated by photoelectrically detecting the chemiluminescence emission released from the absorptive region of the biochemical analysis unit for the second exposure time period is equal to or higher than the saturated value, thereby producing biochemical analysis data of the plurality of the biochemical analysis unit. Therefore, while the conventional method is incapable of producing biochemical analysis data having an excellent quantitative characteristic by using a solid state area sensor to detect chemiluminescence emission released from an absorptive region containing an extremely large amount of a labeling substance which generates chemiluminescence emission when it contacts a chemiluminescent substrate because the intensity of the chemiluminescence emission is extremely high and the number of photons generated by the chemiluminescence emission and entering the photo-electric detecting surface of the solid state area sensor exceeds the dynamic range of the solid state area sensor, the method of present invention can produce biochemical analysis data having an excellent quantitative characteristic even in such a case. On the other hand, while the conventional method cannot easily produce biochemical analysis data having an excellent quantitative characteristic by using a solid state area sensor to photoelectrically detecting chemiluminescence emission because the absorptive region contains only a small amount of the labeling substance which generates chemiluminescence emission when it contacts a chemiluminescent substrate and the intensity of chemiluminescence emission released from the absorptive region is low, the method of the present invention can produce biochemical analysis data having an excellent quantitative characteristic even in such a case because the signal intensity of a digital signal obtained by photoelectrically detecting chemiluminescence emission released from the absorptive region of the biochemical analysis unit for the second exposure time period sufficiently longer than the first exposure time period is adopted as biochemical analysis data of the absorptive region.

In a preferred aspect of the present invention, the data processing means is constituted so as to further produce a correction coefficient based on a ratio of a signal intensity of a digital signal generated by photoelectrically detecting chemiluminescence emission released from an absorptive region of the biochemical analysis unit for the second exposure time period and stored in the memory and whose signal intensity is lower than the saturated value and a signal intensity of a digital signal generated by photoelectrically detecting chemiluminescence emission released from the absorptive region of the biochemical analysis unit for the first exposure time period and stored in the memory, and to multiply a signal intensity of the digital signal generated by photoelectrically detecting chemiluminescence emission released from an absorptive region of the biochemical analysis unit for the second exposure time period and whose signal intensity is equal to or higher than the saturated value by the correction coefficient, thereby producing biochemical analysis data of the absorptive region.

According to this preferred aspect of the present invention, since the data processing means is constituted so as to further produce a correction coefficient based on a ratio of a signal intensity of a digital signal generated by photoelectrically detecting chemiluminescence emission released from an absorptive region of the biochemical analysis unit for the second exposure time period and stored in the memory and whose signal intensity is lower than the saturated value and a signal intensity of a digital signal generated by photoelectrically detecting chemiluminescence emission released from the absorptive region of the biochemical analysis unit for the first exposure time period and stored in the memory, and to multiply a signal intensity of the digital signal generated by photoelectrically detecting chemiluminescence emission released from an absorptive region of the biochemical analysis unit for the second exposure time period and whose signal intensity is equal to or higher than the saturated value by the correction coefficient, thereby producing biochemical analysis data of the absorptive region, the signal intensity of the digital signal generated by photoelectrically detecting chemiluminescence emission released from an absorptive region of the biochemical analysis unit for the first exposure time period, stored in the memory and adopted as biochemical analysis data of the absorptive region of the biochemical analysis unit when the signal intensity of the digital signal generated by photoelectrically detecting chemiluminescence emission released from the absorptive region of the biochemical analysis unit for the second exposure time period and stored in the memory is equal to or higher than the saturated value is corrected so as to be equivalent to that generated by photoelectrically detecting chemiluminescence emission released from an absorptive region of the biochemical analysis unit for the second exposure time period similarly to the signal intensity of the digital signal generated by photoelectrically detecting chemiluminescence emission released from an absorptive region of the biochemical analysis unit for the second exposure time period and stored in the memory and whose signal intensity is lower than the saturated value. Therefore, quantitative analysis can be effected with high accuracy in a desired manner by comparing the signal intensities of the digital signals generated by photoelectrically detecting chemiluminescence emission released from the plurality of absorptive regions of the biochemical analysis unit.

In a further preferred aspect of the present invention, the data processing means is constituted so as to divide a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient.

According to this preferred aspect of the present invention, since the data processing means is constituted so as to divide a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient, it is possible to produce biochemical analysis data having a more excellent quantitative characteristic.

In a preferred aspect of the present invention, each of the plurality of light guide members is formed of at least one optical fiber.

In another preferred aspect of the present invention, each of the plurality of light guide members is formed of an optical fiber bundle constituted by a plurality of optical fibers.

In a preferred aspect of the present invention, the plurality of light guide members are gathered in the vicinity of end portions opposite to the light collecting end portions.

According to this preferred aspect of the present invention, since the plurality of light guide members are gathered in the vicinity of end portions opposite to the light collecting end portions, it is possible to employ a two-dimensional sensor having a small light detecting surface, thereby enabling an apparatus for producing biochemical analysis data to be smaller and lowering cost for manufacturing it.

In a preferred aspect of the present invention, the plurality of light guide members are mounted on a fixing head in the vicinity of the light collecting end portions so that each of the light collecting end portions of the plurality of light guide members is disposed to face one of the plurality of absorptive regions.

In a further preferred aspect of the present invention, the solid state area sensor is constituted by a cooled CCD area sensor.

According to this preferred aspect of the present invention, since the solid state area sensor is constituted by a cooled CCD area sensor, it is possible to collect weak chemiluminescence emission released from the plurality of absorptive regions of the biochemical analysis unit by the plurality of light guide members and photoelectrically detect it for a long time and, therefore, it is possible to detect chemiluminescence emission with sufficiently high sensitivity to produce biochemical analysis data.

In a preferred aspect of the present invention, the biochemical analysis unit includes a substrate formed with a plurality of holes to be spaced apart from each other and the plurality of absorptive regions are formed by causing an absorptive material charged in the plurality of holes formed in the substrate to hold specific binding substances.

In a further preferred aspect of the present invention, the biochemical analysis unit includes a substrate formed with a plurality of through-holes to be spaced apart from each other and the plurality of absorptive regions are formed by causing an absorptive material charged in the plurality of through-holes formed in the substrate to hold specific binding substances.

In a further preferred aspect of the present invention, the plurality of absorptive regions are formed by pressing an absorptive membrane containing an absorptive material into the plurality of through-holes formed in the substrate and causing the absorptive membrane to hold specific binding substances.

In a further preferred aspect of the present invention, the biochemical analysis unit includes a substrate formed with a plurality of recesses to be spaced apart from each other and the plurality of absorptive regions are formed by causing an absorptive material charged in the plurality of recesses formed in the substrate to hold specific binding substances.

In another preferred aspect of the present invention, the biochemical analysis unit includes an absorptive substrate and a substrate formed with a plurality of through-holes to be spaced apart from each other and closely contacted with at least one surface of the absorptive substrate and the plurality of absorptive regions are formed by causing the absorptive substrate in the plurality of through-holes formed in the substrate to hold specific binding substances.

In a preferred aspect of the present invention, the substrate of the biochemical analysis unit has a property of attenuating light energy.

According to this preferred aspect of the present invention, since the substrate of the biochemical analysis unit has a property of attenuating light energy, it is possible to prevent chemiluminescence emission released from the absorptive regions formed in the substrate of the biochemical analysis unit from scattering in the substrate of the biochemical analysis unit and being mixed with each other even in the case of forming the absorptive regions in the substrate of the biochemical analysis unit with high density and selectively hybridizing a substance derived from a living organism and labeled with a labeling substance which generate chemiluminescence emission when it contacts a chemiluminescent substrate. Therefore, it is possible to photoelectrically detect chemiluminescence emission and produce biochemical analysis data having an excellent quantitative characteristic.

In a preferred aspect of the present invention, the substrate of the biochemical analysis unit has a property of reducing the energy of light to 1/5 or less when the light travels in the substrate by a distance equal to that between neighboring absorptive regions.

In a further preferred aspect of the present invention, the substrate of the biochemical analysis unit has a property of reducing the energy of light to 1/10 or less when the light travels in the substrate by a distance equal to that between neighboring absorptive regions.

In a further preferred aspect of the present invention, the substrate of the biochemical analysis unit has a property of reducing the energy of light to 1/50 or less when the light travels in the substrate by a distance equal to that between neighboring absorptive regions.

In a further preferred aspect of the present invention, the substrate of the biochemical analysis unit has a property of reducing the energy of light to 1/100 or less when the light travels in the substrate by a distance equal to that between neighboring absorptive regions.

In a further preferred aspect of the present invention, the substrate of the biochemical analysis unit has a property of reducing the energy of light to 1/500 or less when the light travels in the substrate by a distance equal to that between neighboring absorptive regions.

In a further preferred aspect of the present invention, the substrate of the biochemical analysis unit has a property of reducing the energy of light to 1/1,000 or less when the light travels in the substrate by a distance equal to that between neighboring absorptive regions.

In a preferred aspect of the present invention, the biochemical analysis unit is formed with 10 or more absorptive regions.

In a further preferred aspect of the present invention, the biochemical analysis unit is formed with 50 or more absorptive regions.

In a further preferred aspect of the present invention, the biochemical analysis unit is formed with 100 or more absorptive regions.

In a further preferred aspect of the present invention, the biochemical analysis unit is formed with 500 or more absorptive regions.

In a further preferred aspect of the present invention, the biochemical analysis unit is formed with 1,000 or more absorptive regions.

In a further preferred aspect of the present invention, the biochemical analysis unit is formed with 5,000 or more absorptive regions.

In a further preferred aspect of the present invention, the biochemical analysis unit is formed with 10,000 or more absorptive regions.

In a further preferred aspect of the present invention, the biochemical analysis unit is formed with 50,000 or more absorptive regions.

In a further preferred aspect of the present invention, the biochemical analysis unit is formed with 100,000 or more absorptive regions.

In a preferred aspect of the present invention, each of the plurality of absorptive regions is formed in the biochemical analysis unit to have a size of less than 5 mm$^2$.

In a further preferred aspect of the present invention, each of the plurality of absorptive regions is formed in the biochemical analysis unit to have a size of less than 1 mm$^2$.

In a further preferred aspect of the present invention, each of the plurality of absorptive regions is formed in the biochemical analysis unit to have a size of less than 0.5 mm$^2$.

In a further preferred aspect of the present invention, each of the plurality of absorptive regions is formed in the biochemical analysis unit to have a size of less than 0.1 mm$^2$.

In a further preferred aspect of the present invention, each of the plurality of absorptive regions is formed in the biochemical analysis unit to have a size of less than 0.05 mm$^2$.

In a further preferred aspect of the present invention, each of the plurality of absorptive regions is formed in the biochemical analysis unit to have a size of less than 0.01 mm$^2$.

In a preferred aspect of the present invention, the plurality of absorptive regions are formed in the biochemical analysis unit at a density of 10 or more per cm$^2$.

In a further preferred aspect of the present invention, the plurality of absorptive regions are formed in the biochemical analysis unit at a density of 50 or more per cm$^2$.

In a further preferred aspect of the present invention, the plurality of absorptive regions are formed in the biochemical analysis unit at a density of 100 or more per cm$^2$.

In a further preferred aspect of the present invention, the plurality of absorptive regions are formed in the biochemical analysis unit at a density of 500 or more per cm$^2$.

In a further preferred aspect of the present invention, the plurality of absorptive regions are formed in the biochemical analysis unit at a density of 1,000 or more per cm$^2$.

In a further preferred aspect of the present invention, the plurality of absorptive regions are formed in the biochemical analysis unit at a density of 5,000 or more per cm$^2$.

In a further preferred aspect of the present invention, the plurality of absorptive regions are formed in the biochemical analysis unit at a density of 10,000 or more per cm$^2$.

In a further preferred aspect of the present invention, the plurality of absorptive regions are formed in the biochemical analysis unit at a density of 50,000 or more per cm$^2$.

In a further preferred aspect of the present invention, the plurality of absorptive regions are formed in the biochemical analysis unit at a density of 100,000 or more per cm$^2$.

In a preferred aspect of the present invention, the plurality of absorptive regions are formed in the biochemical analysis unit in a regular pattern.

In a preferred aspect of the present invention, each of the absorptive regions is formed substantially circular in the substrate of the biochemical analysis unit.

In the present invention, the material for forming the substrate of the biochemical analysis unit is preferably capable of attenuating light energy but is not particularly limited. The material for forming the substrate of the biochemical analysis unit may be any type of inorganic compound material or organic compound material and the substrate of the biochemical analysis unit can preferably be formed of a metal material, a ceramic material or a plastic material.

Illustrative examples of inorganic compound materials preferably usable for forming the substrate of the biochemical analysis unit in the present invention include metals such as gold, silver, copper, zinc, aluminum, titanium, tantalum, chromium, iron, nickel, cobalt, lead, tin, selenium and the like; alloys such as brass, stainless steel, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like.

In the present invention, a high molecular compound can preferably be used as an organic compound material preferably usable for forming the substrate of the biochemical analysis unit. Illustrative examples of high molecular compounds preferably usable for forming the substrate of the biochemical analysis unit in the present invention include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Since the capability of attenuating light energy generally increases as scattering and/or absorption of light increases, the substrate of the biochemical analysis unit preferably has absorbance of 0.3 per cm (thickness) or more and more preferably has absorbance of 1 per cm (thickness) or more. The absorbance can be determined by placing an integrating sphere immediately behind a plate-like member having a thickness of T cm, measuring an amount A of transmitted light at a wavelength of probe light or emission light used for measurement by a spectrophotometer, and calculating A/T. In the present invention, a light scattering substance or a light absorbing substance may be added to the substrate of the biochemical analysis unit in order to improve the capability of attenuating light energy. Particles of a material different from a material forming the substrate of the biochemical analysis unit may be preferably used as a light scattering substance and a pigment or dye may be preferably used as a light absorbing substance.

In another preferred aspect of the present invention, the biochemical analysis unit includes an absorptive substrate and the plurality of absorptive regions are formed by causing the absorptive substrate to hold specific binding substances.

In the present invention, a porous material or a fiber material may be preferably used as the absorptive material for forming the absorptive regions or the absorptive substrate of the biochemical analysis unit. The absorptive regions or the absorptive substrate may be formed by combining a porous material and a fiber material.

In the present invention, a porous material for forming the absorptive regions or the absorptive substrate of the biochemical analysis unit may be any type of an organic material or an inorganic material and may be an organic/inorganic composite material.

In the present invention, an organic porous material used for forming the absorptive regions or the absorptive substrate of the biochemical analysis unit is not particularly limited but a carbon porous material such as an activated carbon or a porous material capable of forming a membrane filter is preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof.

In the present invention, an inorganic porous material used for forming the absorptive regions or the absorptive substrate of the biochemical analysis unit is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof.

In the present invention, a fiber material used for forming the absorptive regions or the absorptive substrate of the biochemical analysis unit is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

In the present invention, the absorptive regions of the biochemical analysis unit may be formed using an oxidization process such as an electrolytic process, a plasma process, an arc discharge process or the like; a primer process using a silane coupling agent, titanium coupling agent or the like; and a surface-active agent process or the like.

The above and other objects and features of the present invention will become apparent from the following description made with reference to the accompanying drawings.

**BRIEF DESCRIPTION OF THE DRAWINGS**

Figure 1 is a schematic perspective view showing a biochemical analysis unit used in a method for producing biochemical analysis data which is a preferred embodiment of the present invention.
Figure 2 is a schematic front view showing a spotting device.
Figure 3 is a schematic longitudinal cross sectional view showing a hybridization reaction vessel.
Figure 4 is a schematic cross sectional view showing an apparatus for producing biochemical analysis data which is adapted for reading chemiluminescence data recorded in a number of absorptive regions of a biochemical analysis unit and producing biochemical analysis data.
Figure 5 is a block diagram of a control system, a detection system and a memory system of a cooled CCD area sensor and a control system, a memory system, a display system and an input system of an apparatus for producing biochemical analysis data, which is a preferred embodiment of the present invention.
Figure 6 is a graph showing one example of a signal intensity of a digital signal of each of absorptive regions of a biochemical analysis unit produced by exposing a CCD of a cooled CCD area sensor to chemiluminescence emission for a second exposure time period sufficiently longer than a first exposure time period.
Figure 7 is a graph showing one example of a signal intensity of a digital signal of each of absorptive regions of a biochemical analysis unit produced by exposing a CCD of a cooled CCD area sensor to chemiluminescence emission for a first exposure time period shorter than a second exposure time period.

**DESCRIPTION OF THE PREFERRED EMBODIMENTS**

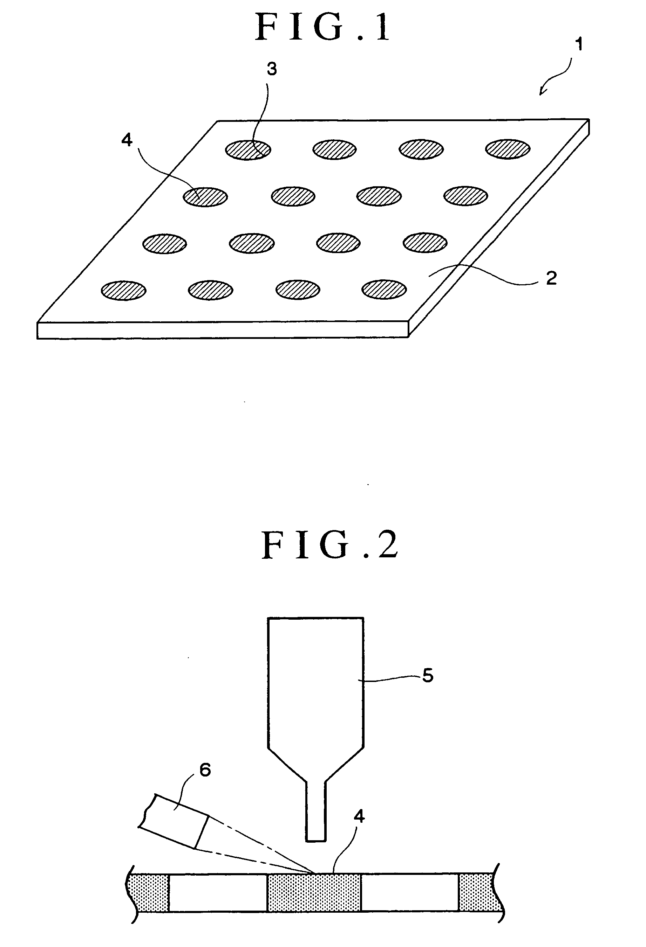

Figure 1 is a schematic perspective view showing a biochemical analysis unit used in a method for producing biochemical analysis data which is a preferred embodiment of the present invention.

As shown in Figure 1, a biochemical analysis unit 1 according to this embodiment includes a substrate 2 formed of aluminum and formed with a number of substantially circular through-holes 3 at a high density, and a number of absorptive regions 4 are dot-like formed by charging nylon-6 in the through-holes 3.

Although not accurately shown in Figure 1, in this embodiment, substantially circular through-holes 3 having a size of about 0.01 mm$^2$ are regularly formed in the manner of a matrix of 120 columns x 160 lines and, therefore, 19,200 absorptive regions 4 are formed.

The absorptive regions 4 are formed by charging nylon-6 in a number of the through-holes 3 in such a manner that the surfaces of the absorptive regions 4 are located at the same height level as that of the substrate 2.

When biochemical analysis is performed, a solution containing specific binding substances such as a plurality of cDNAs whose sequences are known but differ from each other are spotted using a spotting device onto a number of the absorptive regions 4 of the biochemical analysis unit 1 and the specific binding substances are absorbed therein.

Figure 2 is a schematic front view showing a spotting device.

As shown in Figure 2, the spotting device includes an injector 5 for ejecting a solution of specific binding substances toward the biochemical analysis unit 1 and a CCD camera 6 and is constituted so that the solution of specific binding substances such as cDNAs are spotted from the injector 5 when the tip end portion of the injector 5 and the center of the absorptive region 4 into which the solution containing specific binding substances is to be spotted are determined to coincide with each other as a result of viewing them using the CCD camera 6, thereby ensuring that the solution of specific binding substances can be accurately spotted into a number of the absorptive regions 4 of the biochemical analysis unit 1.

Then, a substance derived from a living organism and labeled with a labeling substance which generates chemiluminescence emission when it contacts a chemiluminescent substrate is selectively hybridized with the specific binding substances such as cDNAs absorbed in number of the absorptive regions 4 formed in the substrate 2 of the biochemical analysis unit 1.

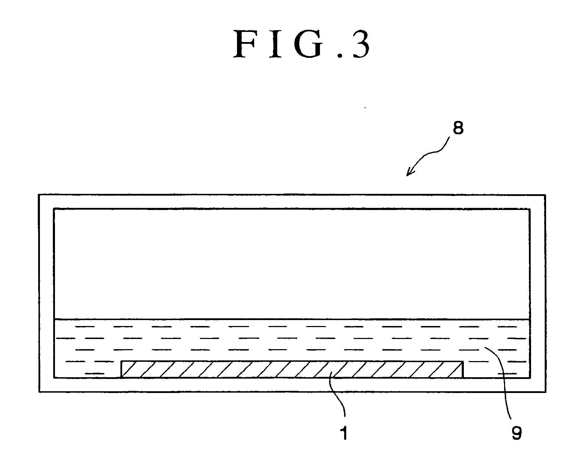

Figure 3 is a schematic longitudinal cross sectional view showing a hybridization reaction vessel.

As shown in Figure 3, a hybridization reaction vessel 8 is formed to have a substantially rectangular cross section and accommodates a hybridization solution 9 containing a substance derived from a living organism and labeled with a labeling substance which generate chemiluminescence emission when it contacts a chemiluminescent substrate as a probe.

When hybridization is to be performed, the biochemical analysis unit 1 containing specific binding substances such as a plurality of cDNAs absorbed in a number of the absorptive regions 4 is accommodated in the hybridization reaction vessel 8.

As a result, a substance derived from a living organism and labeled with a labeling substance which generate chemiluminescence emission when it contacts a chemiluminescent substrate is selectively hybridized with the specific binding substances absorbed in a number of the absorptive regions 4.

Thus, chemiluminescence data of a labeling substance which generates chemiluminescence emission when it contacts a chemiluminescent substrate are recorded in a number of the absorptive regions 4 formed in the biochemical analysis unit 1.

Chemiluminescence data recorded in a number of the absorptive regions 4 in this manner are read by an apparatus for producing biochemical analysis data including a cooled CCD area sensor, thereby producing biochemical analysis data.

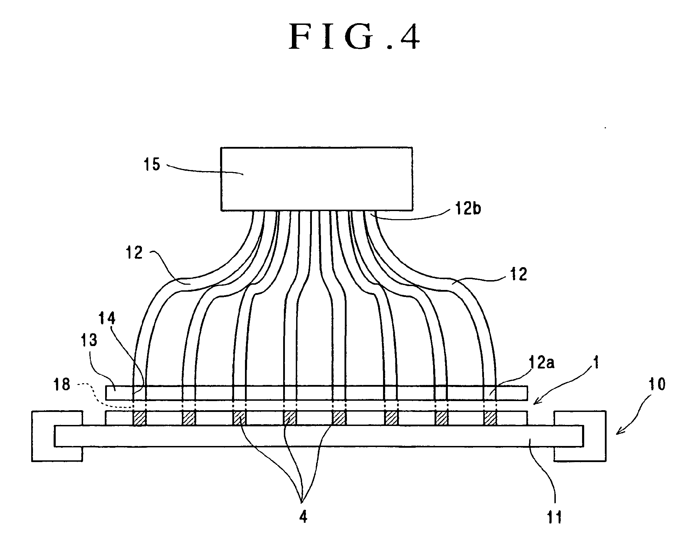

Figure 4 is a schematic cross sectional view showing an apparatus for producing biochemical analysis data which is adapted for reading chemiluminescence data recorded in a number of the absorptive regions of a biochemical analysis unit and producing biochemical analysis data.

As shown in Figure 4, an apparatus for producing biochemical analysis data includes a sample stage 10 provided with a transparent glass plate 11 on which a biochemical analysis unit 1 is to be placed and optical fiber members 12 each having a light collecting end portion 12a facing one of a number of the dot-like absorptive regions 4 of the biochemical analysis unit 1 placed on the sample stage 10 and being located in the vicinity thereof.

In this embodiment, each of the optical fiber members 12 is constituted as a plurality of optical fibers and secured into a through-hole 14 formed in a fixing head 13 in the vicinity of the light collecting end portion 12a so that the light collecting end portion 12a of each of the optical fiber members 12 is positioned in a desired manner.

Further, as shown in Figure 4, the optical fiber members 12 are gathered in the vicinity of end portions 12b opposite to the light collecting end portions 12a.

As shown in Figure 4, each of the optical fiber members 12 is disposed so that end portion 12b thereof opposite to the light collecting end portion 12a faces a photo-electric detecting surface of a cooled CCD area sensor 15.

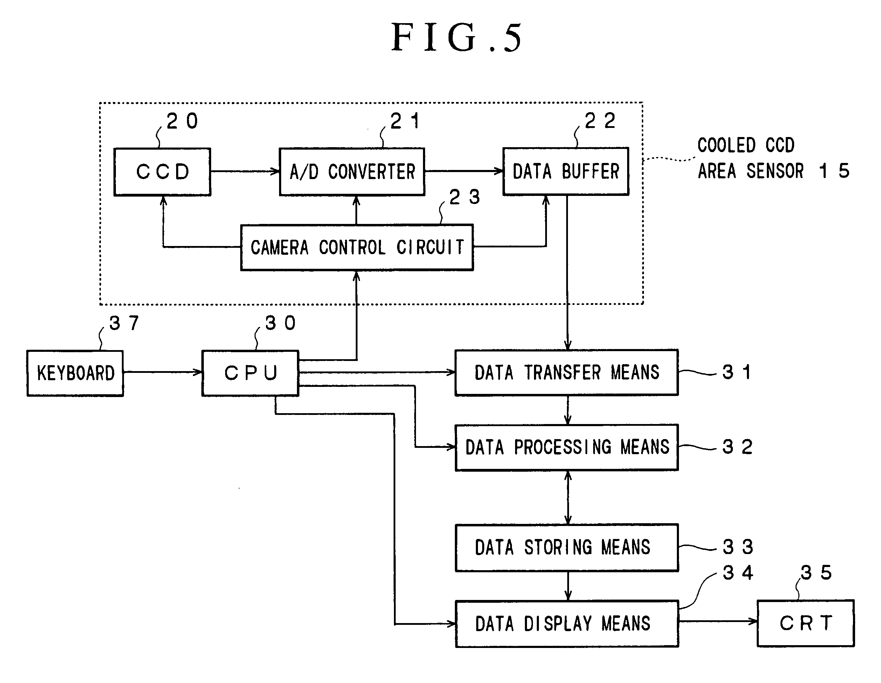

Figure 5 is a block diagram of a control system, a detection system and a memory system of the cooled CCD area sensor 15 and a control system, a memory system, a display system and an input system of the apparatus for producing biochemical analysis data according to this embodiment.

As shown in Figure 5, the cooled CCD area sensor 15 includes a CCD 20, an A/D converter 21 for digitizing analog data produced by the CCD 20 in the form of electric charge, a data buffer 22 for temporarily storing digital signals produced by digitizing analog data by the A/D converter 21 and a camera control circuit 23 for controlling the overall operation of the cooled CCD area sensor 15.

As shown in Figure 5, the apparatus for producing biochemical analysis data according to this embodiment includes a CPU 30 for controlling the overall operation of the cooled CCD area sensor 15, a data transfer means 31 for reading digital signals produced by the cooled CCD area sensor 15 from the data buffer 22, a data processing means 32 for effecting data processing on digital signals read by the data transfer means 31, a data storing means 33 for storing digital signals subjected to data processing by the data processing means 32, a data display means 34 for producing quantitative data based on biochemical analysis data produced by data processing digital signals stored in the data storing means 33 and displaying the quantitative data on the screen of a CRT 35, and a keyboard 37 which can be operated by a user and through which various instruction signals can be input. Based on instruction signals input through the keyboard 37, the CPU 30 is adapted for outputting various signals to the camera control circuit 23 of the cooled CCD area sensor 15.

The thus constituted apparatus for producing biochemical analysis data according to this embodiment reads chemiluminescence data recorded in a number of the absorptive regions formed in the substrate 2 of the biochemical analysis unit 1 and producing biochemical analysis data in the following manner.

The biochemical analysis unit 1 is first placed by a user on the transparent glass plate 11 of the sample stage 10, while in a state of releasing chemiluminescence emission as a result of contact of a labeling substance contained in the absorptive layers 4 formed in the substrate 2 of the biochemical analysis unit 1 and a chemiluminescent substrate.

In this embodiment, guide members (not shown) are provided in the sample stage 10 for ensuring that the biochemical analysis unit 1 is placed on the sample stage 10 so that a number of the absorptive regions 4 face the light collecting end portions 12a of the corresponding optical fiber members 12.

A data production start signal is then input through the keyboard 37 by the user and the data production start signal is input to the CPU 30.

When the CPU 30 receives the data production start signal, it outputs an exposure start signal to the camera control circuit 23 of the cooled CCD area sensor 15, thereby causing the cooled CCD area sensor 15 to start detecting chemiluminescence emission.

Chemiluminescence emission 18 released from each of a number of the absorptive regions 4 of the biochemical analysis unit 1 placed on the sample stage 10 is collected by the light collecting end portion 12a of the corresponding optical fiber member 12 disposed so as to face the absorptive region 4.

In this embodiment, since each of a number of the optical fiber members 12 is secured into the through-hole 14 formed in the fixing head 13 in the vicinity of the light collecting end portion 12a so that the light collecting end portion 12a of each of the optical fiber members 12 faces one of the absorptive region 4 of the biochemical analysis unit 1 placed on the transparent glass plate 11 of the sample stage 10, chemiluminescence emission 18 released from each of the absorptive regions 4 is reliably collected by the light collecting end portion 12a of the corresponding optical fiber member 12.

Chemiluminescence emission 18 collected by the light collecting end portion 12a of a particular optical fiber member 12 is guided by the optical fiber member 12 and impinges onto the photo-electric detecting surface of the CCD 20 of the cooled CCD area sensor 15, thereby forming an image on the photo-electric detecting surface of the CCD 20. The CCD 20 receives light of the thus formed image and accumulates it in the form of electric charges therein.

In this embodiment, since the optical fiber members 12 are gathered in the vicinity of the end portions 12b opposite to the light collecting end portions 12a, even in the case where a number of the optical fiber members 12 are provided correspondingly to a number of the absorptive region 4 formed in the substrate 2 of the biochemical analysis unit 1, it is possible to employ a cooled CCD area sensor 15 provided with a photo-electric detecting surface having a small area. Therefore, it is possible to make an apparatus for producing biochemical analysis data smaller and to lower cost for manufacturing an apparatus for producing biochemical analysis data.

Further, in this embodiment, since the apparatus for producing biochemical analysis data is constituted so as to read chemiluminescence data recorded in each of the absorptive regions 4 formed in the biochemical analysis unit 1 and produce biochemical analysis data, it is not necessary to dispose the end portions 12b of the optical fiber members 12 in the same pattern as that of the light collecting end portions 12a thereof.

When a first exposure time period T1 has passed, the CPU 30 outputs an exposure completion signal to the camera control circuit 23 of the cooled CCD area sensor 15.

When the camera control circuit 23 receives the exposure completion signal from the CPU 30, it transfers analog signals accumulated in the CCD 20 in the form of electric charge during the first exposure time period T1 to the A/D converter 21 to cause the A/D converter 21 to digitize the data, thereby producing a digital signal for each of the absorptive regions 4 of the biochemical analysis unit 1, and to temporarily store the thus produced digital signals in the data buffer 22.

At the same time, the CPU 30 outputs a data transfer signal to the data transfer means 31 to cause it to read out the digital signal of each of the absorptive regions 4 of the biochemical analysis unit 1 from the data buffer 22 of the cooled CCD area sensor 15 and to input them to the data processing means 32.

The data processing means 32 stores the digital signal of each of the absorptive regions 4 of the biochemical analysis unit 1 input from the data transfer means 31 in a memory area for each of the absorptive regions 4 of the biochemical analysis unit 1 in the data storing means 33.

When the CCD 20 of the cooled CCD area sensor 15 has been exposed for the first exposure time period T1 and a digital signal for each of the absorptive regions 4 of the biochemical analysis unit 1 has been produced and stored in the memory area for each of the absorptive regions 4 of the biochemical analysis unit 1 in the data storing means 33 in this manner, the CPU 30 outputs an exposure start signal to the camera control circuit 22 of the cooled CCD area sensor 15, thereby causing the cooled CCD area sensor 15 to again start detecting chemiluminescence emission.

Chemiluminescence emission 18 released from each of a number of the absorptive regions 4 of the biochemical analysis unit 1 placed on the sample stage 10 is collected by the light collecting end portion 12a of the corresponding optical fiber member 12 disposed so as to face the absorptive region 4.

Chemiluminescence emission 18 collected by the light collecting end portion 12a of a particular optical fiber member 12 is guided by the optical fiber member 12 and impinges onto the photo-electric detecting surface of the CCD 20 of the cooled CCD area sensor 15, thereby forming an image on the photo-electric detecting surface of the CCD 20. The CCD 20 receives light of the thus formed image and accumulates it in the form of electric charges therein.

When a second exposure time period T2 longer than the first exposure time period T1 has passed, the CPU 30 outputs an exposure completion signal to the camera control circuit 23 of the cooled CCD area sensor 15.

When the camera control circuit 23 receives the exposure completion signal from the CPU 30, it transfers analog signals accumulated in the CCD 20 in the form of electric charge during the second exposure time period T2 to the A/D converter 21 to cause the A/D converter 21 to digitize the data, thereby producing a digital signal for each of the absorptive regions 4 of the biochemical analysis unit 1, and to temporarily store the thus produced digital signals in the data buffer 22.

At the same time, the CPU 30 outputs a data transfer signal to the data transfer means 31 to cause it to read out the digital signal of each of the absorptive regions 4 of the biochemical analysis unit 1 from the data buffer 22 of the cooled CCD area sensor 15 and to input them to the data processing means 32.

The data processing means 32 stores the digital signal of each of the absorptive regions 4 of the biochemical analysis unit 1 input from the data transfer means 31 in a memory area for each of the absorptive regions 4 of the biochemical analysis unit 1 in the data storing means 33.

When the CCD 20 of the cooled CCD area sensor 15 has been exposed for the second exposure time period T2 longer than the first exposure time period T1 and a digital signal for each of the absorptive regions 4 of the biochemical analysis unit 1 has been produced and stored in the memory area for each of the absorptive regions 4 of the biochemical analysis unit 1 in the data storing means 33 in this manner, the CPU 30 outputs a data processing signal to the data processing means 32.

When the data processing signal is input from the CPU 30, the data processing means 32 reads from the data storing means 33 the digital signal for each of the absorptive regions 4 of the biochemical analysis unit 1 produced by exposing the CCD 20 of the cooled CCD area sensor 15 for the second exposure time period T2.

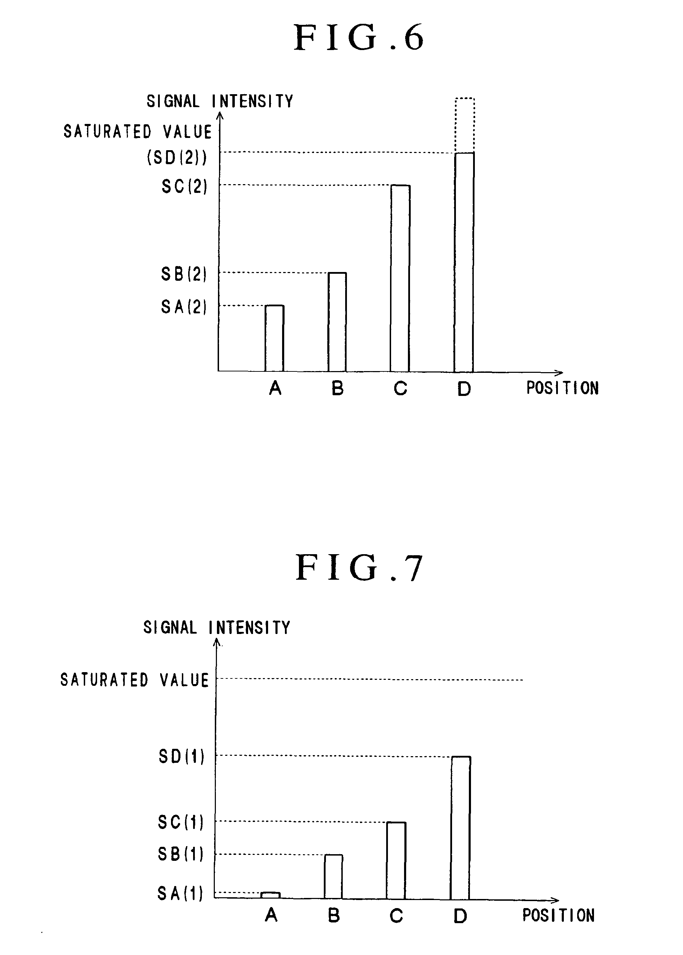

Figure 6 is a graph showing one example of a signal intensity of a digital signal of each of the absorptive regions 4 of the biochemical analysis unit 1 produced by exposing the CCD 20 of the cooled CCD area sensor 15 to chemiluminescence emission 18 for the second exposure time period T2 sufficiently longer than the first exposure time period T1.

In this embodiment, since the second exposure time period T2 is set to be sufficiently longer than the first exposure time period T1, the digital signal produced by photoelectrically detecting chemiluminescence emission 18 having a low intensity and released from an absorptive region 4 containing a small amount of a labeling substance which generates chemiluminescence emission 18 when it contacts a chemiluminescent substrate with the cooled CCD area sensor 15 has a signal intensity which can be quantitatively analyzed. On the other hand, however, when chemiluminescence emission 18 released from an absorptive region 4 containing a large amount of a labeling substance which generates chemiluminescence emission 18 when it contacts a chemiluminescent substrate is photoelectrically detected by the CCD 20 of the cooled CCD area sensor 15, since the intensity of the chemiluminescence emission 18 is high, the number of photons generated by the chemiluminescence emission 18 and entering the photo-electric detecting surface of the CCD 20 of the cooled CCD area sensor 15 may exceed the upper limit of the dynamic range of the CCD 20 and if the number of photons generated by the chemiluminescence emission 18 and entering the photo-electric detecting surface of the CCD 20 of the cooled CCD area sensor 15 solid state area sensor exceeds the upper limit of the dynamic range of the CCD 20, the digital signal is saturated and has no quantitative characteristic.

In the example shown in Figure 6, the signal intensity SA(2) of a digital signal obtained by photoelectrically detecting chemiluminescence emission 18 released from an absorptive region 4 located at a position A of the substrate 2 of the biochemical analysis unit 1 and containing a small amount of the labeling substance which generates chemiluminescence emission 18 when it contacts a chemiluminescent substrate, the signal intensity SB(2) of a digital signal obtained by photoelectrically detecting chemiluminescence emission 18 released from an absorptive region 4 located at a position B of the substrate 2 of the biochemical analysis unit 1 and containing more but not a large amount of the labeling substance which generates chemiluminescence emission 18 when it contacts a chemiluminescent substrate and the signal intensity SC(2) of a digital signal obtained by photoelectrically detecting chemiluminescence emission 18 released from an absorptive region 4 located at a position C of the substrate 2 of the biochemical analysis unit 1 and containing even more but still not such a large amount of the labeling substance which generates chemiluminescence emission 18 when it contacts a chemiluminescent substrate are lower than the saturated value and, therefore, it can be considered that the signal intensities of the digital signals have an excellent quantitative characteristic. On the other hand, the signal intensity SD(2) of a digital signal obtained by photoelectrically detecting chemiluminescence emission 18 released from an absorptive region 4 located at a position D of the substrate 2 of the biochemical analysis unit 1 and containing an extremely large amount of the labeling substance which generates chemiluminescence emission 18 when it contacts a chemiluminescent substrate exceeds the saturated value since the intensity of chemiluminescence emission 18 released therefrom is high and the signal intensity of the digital signal has no quantitative characteristic.

Therefore, the data processing means 32 employs the signal intensity of a digital signal whose signal intensity is lower than the saturated value as biochemical analysis data representing an amount of the labeling substance which generates chemiluminescence emission 18 when it contacts a chemiluminescent substrate contained in the absorptive region 4 of the biochemical analysis unit 1 and stores it in the memory area for each of the absorptive regions 4 of the biochemical analysis unit 1 in the data storing means 33. On the other hand, the data processing means 32 neither employs the signal intensity of a digital signal whose signal intensity is equal to or higher than the saturated value as biochemical analysis data representing an amount of the labeling substance which generates chemiluminescence emission 18 when it contacts a chemiluminescent substrate contained in the absorptive region 4 of the biochemical analysis unit 1 nor stores it in the data storing means 33.

In the example shown in Figure 6, the data processing means 32 employs the signal intensity SA(2) of a digital signal obtained by photoelectrically detecting chemiluminescence emission 18 released from an absorptive region 4 located at the position A of the substrate 2 of the biochemical analysis unit 1 and containing a small amount of the labeling substance which generates chemiluminescence emission 18 when it contacts a chemiluminescent substrate, the signal intensity SB(2) of a digital signal obtained by photoelectrically detecting chemiluminescence emission 18 released from an absorptive region 4 located at the position B of the substrate 2 of the biochemical analysis unit 1 and containing more but not a large amount of the labeling substance which generates chemiluminescence emission 18 when it contacts a chemiluminescent substrate and the signal intensity SC(2) of a digital signal obtained by photoelectrically detecting chemiluminescence emission 18 released from an absorptive region 4 located at the position C of the substrate 2 of the biochemical analysis unit 1 and containing even more but still not such a large amount of the labeling substance which generates chemiluminescence emission 18 when it contacts a chemiluminescent substrate as biochemical analysis data SA, SB and SC representing an amount of the labeling substance which generates chemiluminescence emission 18 when it contacts a chemiluminescent substrate contained in the absorptive region 4 of the biochemical analysis unit 1 and stores them in the memory areas for each of the absorptive regions 4 of the biochemical analysis unit 1 in the data storing means 33. On the other hand, the data processing means 32 neither employs the signal intensity SD(2) of a digital signal obtained by photoelectrically detecting chemiluminescence emission 18 released from an absorptive region 4 located at the position D of the substrate 2 of the biochemical analysis unit 1 and containing an extremely large amount of the labeling substance which generates chemiluminescence emission 18 when it contacts a chemiluminescent substrate nor stores it in the data storing means 33.

The data processing means 32 next reads from the data storing means 33 the digital signal for each of the absorptive regions 4 of the biochemical analysis unit 1 produced by exposing the CCD 20 of the cooled CCD area sensor 15 for the first exposure time period T1 shorter than the second exposure time period T2.

Figure 7 is a graph showing one example of the signal intensity of a digital signal of each of the absorptive regions 4 of the biochemical analysis unit 1 produced by exposing the CCD 20 of the cooled CCD area sensor 15 to chemiluminescence emission 18 for the first exposure time period T1 shorter than the second exposure time period sufficiently longer than a first exposure time period.

In this embodiment, since the first exposure time period T1 is set to be sufficiently shorter than the second exposure time period T2, the signal intensity of a digital signal produced by photoelectrically detecting chemiluminescence emission 18 released from an absorptive region 4 containing a small amount of a labeling substance which generates chemiluminescence emission 18 when it contacts a chemiluminescent substrate with the cooled CCD area sensor 15 is low and it is therefore difficult to produce biochemical analysis data having an excellent quantitative characteristic by detecting chemiluminescence emission 18 with the CCD 20 of the cooled CCD area sensor 15. On the other hand, however, when chemiluminescence emission 18 released from an absorptive region 4 containing an extremely large amount of a labeling substance which generates chemiluminescence emission 18 when it contacts a chemiluminescent substrate and having a high intensity is photoelectrically detected by the CCD 20 of the cooled CCD area sensor 15, it is possible to effectively prevent the number of photons generated by the chemiluminescence emission and entering the photo-electric detecting surface of the CCD 20 of the cooled CCD area sensor 15 from exceeding the upper limit of the dynamic range of the CCD 20 and it is therefore possible to produce biochemical analysis data having an excellent quantitative characteristic by detecting chemiluminescence emission 18 released from an absorptive region 4 containing an extremely large amount of a labeling substance which generates chemiluminescence emission 18 when it contacts a chemiluminescent substrate and having a high intensity with the CCD 20 of the cooled CCD area sensor 15.

In the example shown in Figure 7, the signal intensity SA(1) of a digital signal obtained by photoelectrically detecting for the first exposure time period T1 chemiluminescence emission 18 released from an absorptive region 4 located at the position A of the substrate 2 of the biochemical analysis unit 1 and containing a small amount of the labeling substance which generates chemiluminescence emission 18 when it contacts a chemiluminescent substrate is much lower than the signal intensity SA(2) of a digital signal obtained by photoelectrically detecting for the second exposure time period T2 chemiluminescence emission 18 released from an absorptive region 4 located at the position A of the substrate 2 of the biochemical analysis unit 1 and containing a small amount of the labeling substance which generates chemiluminescence emission 18 when it contacts a chemiluminescent substrate and it can be considered that the quantitative characteristic thereof is extremely low due to noise. On the other hand, the signal intensity SB(1) of a digital signal obtained by photoelectrically detecting for the first exposure time period T1 chemiluminescence emission 18 released from an absorptive region 4 located at the position B of the substrate 2 of the biochemical analysis unit 1 and containing more but not a large amount of the labeling substance which generates chemiluminescence emission 18 when it contacts a chemiluminescent substrate and the signal intensity SC(1) of a digital signal obtained by photoelectrically detecting for the first exposure time period T1 chemiluminescence emission 18 released from an absorptive region 4 located at the position CB of the substrate 2 of the biochemical analysis unit 1 and containing even more but still not such a large amount of the labeling substance which generates chemiluminescence emission 18 when it contacts a chemiluminescent substrate are lower than the signal intensity SB(2) of a digital signal obtained by photoelectrically detecting for the second exposure time period T2 chemiluminescence emission 18 released from an absorptive region 4 located at the position B of the substrate 2 of the biochemical analysis unit 1 and containing more but not a large amount of the labeling substance which generates chemiluminescence emission 18 when it contacts a chemiluminescent substrate and the signal intensity SC(2) of a digital signal obtained by photoelectrically detecting for the second exposure time period T2 chemiluminescence emission 18 released from an absorptive region 4 located at the position C of the substrate 2 of the biochemical analysis unit 1 and containing even more but still not such a large amount of the labeling substance which generates chemiluminescence emission 18 when it contacts a chemiluminescent substrate and it can be considered that the signal intensity SB(2) of a digital signal obtained by photoelectrically detecting for the second exposure time period T2 chemiluminescence emission 18 released from an absorptive region 4 located at the position B of the substrate 2 of the biochemical analysis unit 1 and containing more but not a large amount of the labeling substance which generates chemiluminescence emission 18 when it contacts a chemiluminescent substrate and the signal intensity SC(2) of a digital signal obtained by photoelectrically detecting for the second exposure time period T2 chemiluminescence emission 18 released from an absorptive region 4 located at the position C of the substrate 2 of the biochemical analysis unit 1 and containing even more but still not such a large amount of the labeling substance which generates chemiluminescence emission 18 when it contacts a chemiluminescent substrate have a more excellent quantitative characteristic. To the contrary, the signal intensity SD(1) of a digital signal obtained by photoelectrically detecting for the first exposure time period T1 chemiluminescence emission 18 released from an absorptive region 4 located at the position D of the substrate 2 of the biochemical analysis unit 1 and containing an extremely large amount of the labeling substance which generates chemiluminescence emission 18 when it contacts a chemiluminescent substrate is lower than the saturated value and has an excellent quantitative characteristic since the intensity of chemiluminescence emission 18 released therefrom is high.

Therefore, when the signal intensity of digital data produced by photoelectrically detecting chemiluminescence emission 18 released from an absorptive region 4 of the biochemical analysis unit 1 for the second exposure time period T2 is equal to or higher than the saturated value, the data processing means 32 selects the signal intensity of the digital signal generated by photoelectrically detecting chemiluminescence emission 18 released from the absorptive region 4 of the biochemical analysis unit 1 for the first exposure time period T1 and whose signal intensity is lower than the saturated value as biochemical analysis data representing an amount of the labeling substance which generates chemiluminescence emission 18 when it contacts a chemiluminescent substrate contained in the absorptive region 4 of the biochemical analysis unit 1, i.e., in the examples shown in Figures 6 and 7, it selects the signal intensity SD(1) of the digital signal produced by photoelectrically detecting for the first exposure time period T1 chemiluminescence emission 18 released from an absorptive region 4 located at the position D of the substrate 2 of the biochemical analysis unit 1 and containing an extremely large amount of the labeling substance which generates chemiluminescence emission 18 when it contacts a chemiluminescent substrate.

However, in the case where the signal intensity of the digital signal generated by photoelectrically detecting chemiluminescence emission 18 released from an absorptive region 4 of the biochemical analysis unit 1 for the first exposure time period T1 and whose signal intensity is lower than the saturated value is selected as biochemical analysis data representing an amount of the labeling substance which generates chemiluminescence emission 18 when it contacts a chemiluminescent substrate contained in the absorptive region 4 of the biochemical analysis unit 1 in this manner when the signal intensity of a digital data produced by photoelectrically detecting chemiluminescence emission 18 released from an absorptive region 4 of the biochemical analysis unit 1 for the second exposure time period T2 is equal to or higher than the saturated value, in the case where the signal intensity SD(1) of the digital signal produced by photoelectrically detecting for the first exposure time period T1 chemiluminescence emission 18 released from an absorptive region 4 located at the position D of the substrate 2 of the biochemical analysis unit 1 and containing an extremely large amount of the labeling substance which generates chemiluminescence emission 18 when it contacts a chemiluminescent substrate is selected as biochemical analysis data representing an amount of the labeling substance which generates chemiluminescence emission 18 when it contacts a chemiluminescent substrate contained in the absorptive region 4 of the biochemical analysis unit 1 in the examples shown in Figures 6 and 7, since the exposure time period is different from that in the case of selecting the signal intensity of a digital data produced by photoelectrically detecting chemiluminescence emission 18 released from an absorptive region 4 of the biochemical analysis unit 1 for the second exposure time period T2 as biochemical analysis data representing an amount of the labeling substance which generates chemiluminescence emission 18 when it contacts a chemiluminescent substrate contained in the absorptive region 4 of the biochemical analysis unit 1, quantitative analysis cannot be performed by comparing the absolute values of the signal intensities of digital signals.

Therefore, the data processing means 32 reads the digital signal whose signal intensity is highest among signal intensities of digital signals produced by photoelectrically detecting chemiluminescence emission 18 for the second exposure time period T2, adopted as biochemical analysis data representing amounts of the labeling substance which generates chemiluminescence emission 18 when it contacts a chemiluminescent substrate contained in the absorptive regions 4 of the biochemical analysis unit 1 and stored in the data storing means 33 and divides the thus read signal intensity of the digital signal by the signal intensity of the digital signal obtained by photoelectrically detecting chemiluminescence emission 18 released from the same absorptive region 4 for the first exposure time period T1, thereby producing a correction coefficient K. The data processing means 32 then multiplies the signal intensity of the digital signal generated by photoelectrically detecting chemiluminescence emission 18 released from the absorptive region 4 of the biochemical analysis unit 1 for the first exposure time period T1 and whose signal intensity is lower than the saturated value although the signal intensity of the digital signal obtained by photoelectrically detecting chemiluminescence emission 18 released from the absorptive region 4 for the second exposure time period T2 is equal to or higher than the saturated value by the correction coefficient K, defines the thus obtained signal intensity as biochemical analysis data representing an amount of the labeling substance which generates chemiluminescence emission 18 when it contacts a chemiluminescent substrate contained in the absorptive region 4 of the biochemical analysis unit 1 and stores it in the memory area assigned for each of the absorptive regions 4 of the biochemical analysis unit 1 in the data storing means 33.

In the examples shown in Figures 6 and 7, since the highest signal intensity among signal intensities of digital signals produced by photoelectrically detecting chemiluminescence emission 18 for the second exposure time period T2 and adopted as biochemical analysis data representing amounts of the labeling substance which generates chemiluminescence emission 18 when it contacts a chemiluminescent substrate contained in the absorptive regions 4 of the biochemical analysis unit 1 is the signal intensity SC(2), the data processing means 32 reads the signal intensity SC(2) of the digital signal stored in the data storing means 33 and, in accordance with the following formula, divides the signal intensity SC(2) by the signal intensity SC(1) of the digital signal obtained by photoelectrically detecting chemiluminescence emission 18 released from the same absorptive region 4 for the first exposure time period T1, thereby producing a correction coefficient K.

$$K = SC(2) / SC(1)$$

The data processing means 32 then determines biochemical analysis data SD representing an amount of the labeling substance which generates chemiluminescence emission 18 when it contacts a chemiluminescent substrate contained in the absorptive region 4 located at the position D of the biochemical analysis unit 1 using the thus produced correction coefficient K in accordance with the following formula.

$$SD = SD(1) \times K$$

As described above, biochemical analysis data representing amounts of the labeling substance which generates chemiluminescence emission 18 when it contacts a chemiluminescent substrate contained in a number of the absorptive region 4 of the biochemical analysis unit 1 are produced.

According to the above-described embodiment, since each of a number of the optical fiber members 12 is secured into the through-hole 14 formed in the fixing head 13 in the vicinity of the light collecting end portion 12a so that the light collecting end portion 12a of each of the optical fiber members 12 faces one of the absorptive region 4 of the biochemical analysis unit 1 placed on the transparent glass plate 11 of the sample stage 10, chemiluminescence emission 18 released from each of the absorptive regions 4 of the biochemical analysis unit 1 is reliably collected by the light collecting end portion 12a of the corresponding optical fiber member 12 and, therefore, the efficiency for collecting chemiluminescence emission 18 can be markedly improved.

On the other hand, in the case where chemiluminescence emission 18 released from each of the absorptive regions 4 of the biochemical analysis unit 1 is led by the plurality of optical fiber members 12 to the CCD 20 of the cooled area sensor 15 for improving the efficiency for collecting chemiluminescence emission 18, when chemiluminescence emission 18 released from an absorptive region 4 containing a large amount of a labeling substance which generates chemiluminescence emission 18 when it contacts a chemiluminescent substrate is photoelectrically detected by the CCD 20 of the cooled CCD area sensor 15, since the intensity of the chemiluminescence emission 18 is high, the number of photons generated by the chemiluminescence emission 18 and entering the photo-electric detecting surface of the CCD 20 of the cooled CCD area sensor 15 may exceed the upper limit of the dynamic range of the CCD 20. However, according to the above-described embodiment, when the signal intensity SD(2) of the digital data produced by photoelectrically detecting chemiluminescence emission 18 released from an absorptive region 4 of the biochemical analysis unit 1 for the second exposure time period T2 sufficiently longer than the first exposure time period T1 is equal to or higher than the saturated value, the signal intensity SD(1) of the digital signal generated by photoelectrically detecting chemiluminescence emission 18 released from an absorptive region 4 of the biochemical analysis unit 1 for the first exposure time period T1 sufficiently shorter than the second exposure time period T2 and whose signal intensity is lower than the saturated value is selected as biochemical analysis data representing an amount of the labeling substance which generates chemiluminescence emission 18 when it contacts a chemiluminescent substrate contained in the absorptive region 4 of the biochemical analysis unit 1. Therefore, even in the case where the absorptive region 4 of the biochemical analysis unit 1 contains an extremely large amount of the labeling substance which generates chemiluminescence emission 18 when it contacts a chemiluminescent substrate and the intensity of chemiluminescence emission 18 is very high, so that the number of photons generated by the chemiluminescence emission 18 and entering the photo-electric detecting surface of the CCD 20 of the cooled CCD area sensor exceeds the upper limit of the dynamic range of the CCD 20 owing to the improved collection efficiency of the chemiluminescence emission 18, which would make it impossible for the conventional method to produce biochemical analysis data having an excellent quantitative characteristic, the above-described embodiment can nevertheless produce biochemical analysis data having an excellent quantitative characteristic.

Further, according to the above-described embodiment, biochemical analysis data representing an amount of the labeling substance which generates chemiluminescence emission 18 when it contacts a chemiluminescent substrate contained in the absorptive region 4 of the biochemical analysis unit 1 are determined by multiplying the thus selected biochemical analysis data by the correction coefficient K produced by dividing the signal intensity SC(2) of the digital signal which is highest among the signal intensities of the digital signals produced by photoelectrically detecting chemiluminescence emission 18 released from the absorptive regions 4 of the biochemical analysis unit 1 for the second exposure time period T2, are selected as biochemical analysis data representing an amount of the labeling substance which generates chemiluminescence emission 18 when it contacts a chemiluminescent substrate contained in the absorptive regions 4 of the biochemical analysis unit 1 and are stored in the data storing means 33 by the signal intensity SC(1) of the digital signal produced by photoelectrically detecting chemiluminescence emission 18 released from the same absorptive region 4 of the biochemical analysis unit 1 for the first exposure time period T1. Therefore, quantitative analysis can be performed by comparing biochemical analysis data of a number of the absorptive regions 4 of the biochemical analysis unit 1, notwithstanding that the biochemical analysis data representing the amount of the labeling substance which generates chemiluminescence emission 18 when it contacts a chemiluminescent substrate contained in the absorptive regions 4 of the biochemical analysis unit 1 have been produced by varying the exposure time period of the CCD 20 of the cooled CCD area sensor 15 depending upon the amount of the labeling substance which generates chemiluminescence emission 18 when it contacts a chemiluminescent substrate contained in a number of the absorptive regions 4 of the biochemical analysis unit 1. Moreover, since the correction coefficient K is produced by dividing the signal intensity SC(2) of the digital signal which is the highest and has a most reliable quantitative characteristic among the signal intensities of the digital signals which have been produced by photoelectrically detecting chemiluminescence emission 18 released from the absorptive regions 4 of the biochemical analysis unit 1 for the second exposure time period T2, are selected as biochemical analysis data representing an amount of the labeling substance which generates chemiluminescence emission 18 when it contacts a chemiluminescent substrate contained in the absorptive regions 4 of the biochemical analysis unit 1 and are stored in the data storing means 33 by the signal intensity SC(1) of the digital signal produced by photoelectrically detecting chemiluminescence emission 18 released from the same absorptive region 4 of the biochemical analysis unit 1 for the first exposure time period T1, the biochemical analysis data can be corrected without degrading the quantitative characteristic of the biochemical analysis data to perform quantitative analysis.

Furthermore, according to the above-described embodiment, the signal intensities SA(2), SB(2) and SC(2) of the digital signals obtained by photoelectrically detecting chemiluminescence emission 18 released from the absorptive regions 4 of the biochemical analysis unit 1 for the second exposure time period T2 sufficiently longer than the first exposure time period T1 with the cooled CCD area sensor 15 are respectively defined as biochemical analysis data of the absorptive region 4 containing a small amount of the labeling substance which generates chemiluminescence emission 18 when it contacts a chemiluminescent substrate, biochemical analysis data of the absorptive region 4 containing more but not a large amount of the labeling substance which generates chemiluminescence emission 18 when it contacts a chemiluminescent substrate and biochemical analysis data of the absorptive region 4 containing even more but still not such a large amount of the labeling substance which generates chemiluminescence emission 18 when it contacts a chemiluminescent substrate. Therefore even in a case where it is extremely difficult for the conventional method to produce biochemical analysis data having an excellent quantitative characteristic by photoelectrically detecting chemiluminescence emission 18 released from the absorptive regions 4 of the biochemical analysis unit 1 with the cooled CCD area sensor 15 because the intensity of the chemiluminescence emission 18 is too low, a case where it is extremely difficult for the conventional method to produce biochemical analysis data having an excellent quantitative characteristic by photoelectrically detecting chemiluminescence emission 18 released from the absorptive regions 4 of the biochemical analysis unit 1 with the cooled CCD area sensor 15 because the intensity of the chemiluminescence emission 18 is low, and a case where it is extremely difficult for the conventional method to produce biochemical analysis data having an excellent quantitative characteristic by photoelectrically detecting chemiluminescence emission 18 released from the absorptive regions 4 of the biochemical analysis unit 1 with the cooled CCD area sensor 15 because the intensity of the chemiluminescence emission 18 is not sufficiently high, the above-described embodiment can nevertheless produce biochemical analysis data having an excellent quantitative characteristic by photoelectrically detecting chemiluminescence emission 18 with the cooled CCD area sensor 15.

Moreover, the intensity of chemiluminescence emission 18 becomes lower with the lapse of time and, therefore, the quantitative characteristic of biochemical analysis data produced by photoelectrically detecting chemiluminescence emission 18 becomes lower with the lapse of time. However, according to the above-described embodiment, digital signals for the respective absorptive regions 4 of the biochemical analysis unit 1 are first produced by photoelectrically detecting chemiluminescence emission 18 released from the absorptive regions 4 of the biochemical analysis unit 1 for the first exposure time period T1 sufficiently shorter than the second exposure time period T2 and digital signals for the respective absorptive regions 4 of the biochemical analysis unit 1 are then produced by photoelectrically detecting chemiluminescence emission 18 released from the absorptive regions 4 of the biochemical analysis unit 1 for the second exposure time period T2 sufficiently longer than the first exposure time period T1. Therefore, since the digital signals produced by photoelectrically detecting chemiluminescence emission 18 released from the absorptive regions 4 of the biochemical analysis unit 1 for the second exposure time period T2 sufficiently longer than the first exposure time period T1 have high signal intensities, it is possible to effectively prevent the quantitative characteristic of biochemical analysis data from being lowered due to decrease of the intensity of chemiluminescence emission 18 with the lapse of time.

Further, according to the above-described embodiment, since the substrate 2 of the biochemical analysis unit 1 is formed of aluminum and has a property capable of attenuating light energy, it is possible to effectively prevent chemiluminescence emission 18 released from the neighboring absorptive regions 4 from scattering and mixing with each other and it is therefore possible to effectively prevent noise caused by the scattering of chemiluminescence emission 18 from being generated in biochemical analysis data produced by reading chemiluminescence data recorded in a number of the absorptive regions 4 of the biochemical analysis unit 1.

For example, in the above-described embodiment, although chemiluminescence data are recorded in a number of the absorptive regions 4 of the biochemical analysis unit 1 by hybridizing a substance derived from a living organism and labeled with the labeling substance which generates chemiluminescence emission 18 when it contacts a chemiluminescent substrate with the specific binding substances such as cDNAs fixed in a number of the absorptive regions 4 of the biochemical analysis unit 1, chemiluminescence data may be recorded in a number of the absorptive regions 4 of the biochemical analysis unit 1 by hybridizing a substance derived from a living organism and labeled with a hapten with the specific binding substances such as cDNAs fixed in a number of the absorptive regions 4 of the biochemical analysis unit 1 and further binding an antibody for the hapten labeled with an enzyme which generates chemiluminescence emission when it contacts a chemiluminescent substrate with the hapten labeling the substance derived from a living organism by an antigen-antibody reaction.

Illustrative examples of the combination of the hapten and the antibody include digoxigenin and anti-digoxigenin antibody, theophylline and anti-theophylline antibody, fluorosein and anti-fluorosein antibody, and the like. Further, the combination of biotin and avidin, antigen and antibody may be utilized instead of the combination of hapten and antibody.

Further, in the above-described embodiment, the correction coefficient K is produced by dividing the signal intensity SC(2) of the digital signal which is the highest among signal intensities of digital signals that are produced by photoelectrically detecting chemiluminescence emission 18 for the second exposure time period T2, are adopted as biochemical analysis data representing amounts of the labeling substance which generates chemiluminescence emission 18 when it contacts a chemiluminescent substrate contained in the absorptive regions 4 of the biochemical analysis unit 1 and are stored in the data storing means 33 by the signal intensity SC(1) of a digital signal obtained by photoelectrically detecting chemiluminescence emission 18 released from the same absorptive region 4 for the first exposure time period T1. However, it is not absolutely necessary to produce the correction coefficient K by dividing the signal intensity SC(2) of the digital signal which is the highest among signal intensities of digital signals produced by photoelectrically detecting chemiluminescence emission 18 for the second exposure time period T2, are adopted as biochemical analysis data representing amounts of the labeling substance which generates chemiluminescence emission 18 when it contacts a chemiluminescent substrate contained in the absorptive regions 4 of the biochemical analysis unit 1 and are stored in the data storing means 33 by the signal intensity SC(1) of a digital signal obtained by photoelectrically detecting chemiluminescence emission 18 released from the same absorptive region 4 for the first exposure time period T1, and the correction coefficient K may instead be produced by dividing the signal intensities of digital signals that are produced by photoelectrically detecting chemiluminescence emission 18 for the second exposure time period T2, are adopted as biochemical analysis data representing amounts of the labeling substance which generates chemiluminescence emission 18 when it contacts a chemiluminescent substrate contained in the absorptive regions 4 of the biochemical analysis unit 1 and are stored in the data storing means 33 by the signal intensities of digital signals obtained by photoelectrically detecting chemiluminescence emission 18 released from the corresponding absorptive regions 4 for the first exposure time period T1 and averaging the thus calculated values. Moreover, the correction coefficient K may be produced by dividing the signal intensities of digital signals which are equal to or higher than a predetermined value among signal intensities of digital signals that are produced by photoelectrically detecting chemiluminescence emission 18 for the second exposure time period T2, are adopted as biochemical analysis data representing amounts of the labeling substance which generates chemiluminescence emission 18 when it contacts a chemiluminescent substrate contained in the absorptive regions 4 of the biochemical analysis unit 1 and are stored in the data storing means 33 by the signal intensities of digital signals obtained by photoelectrically detecting chemiluminescence emission 18 released from the corresponding absorptive regions 4 for the first exposure time period T1 and averaging the thus calculated values.

Furthermore, in the above-described embodiment, although 19,200 substantially circular absorptive regions 4 each having a size of about 0.01 mm$^2$ are regularly formed in the manner of a matrix in the substrate 2 of the biochemical analysis unit 1, the shape of each of the absorptive regions 4 is not limited to substantially a circular shape but may be formed in an arbitrary shape, for example, a rectangular shape.

Moreover, in the above-described embodiment, although 19,200 substantially circular absorptive regions 4 each having a size of about 0.01 mm$^2$ are regularly formed in the manner of a matrix in the substrate 2 of the biochemical analysis unit 1, the number or size of the absorptive regions 4 may be arbitrarily selected in accordance with the purpose. Preferably, 10 or more of the absorptive regions 4 having a size of 5 cm$^2$ or less are formed in the substrate 2 at a density of 10/ cm$^2$ or greater.

Further, in the above-described embodiment, although 19,200 substantially circular absorptive regions 4 each having a size of about 0.01 mm$^2$ are regularly formed in the manner of a matrix in the substrate 2 of the biochemical analysis unit 1, it is not absolutely necessary to form a number of the absorptive regions 4 in a regular pattern in the substrate 2 of the biochemical analysis unit 1.

Furthermore, in the above-described embodiment, the biochemical analysis unit 1 includes a number of the absorptive regions 4 formed by charging nylon-6 in a number of the through-holes 3 formed in the substrate 2 made of aluminum. However, it is not absolutely necessary to form a number of the absorptive regions 4 of the biochemical analysis unit 1 of nylon-6 but a number of the absorptive regions 4 of the biochemical analysis unit 1 may be formed of a porous material capable of forming a membrane filter other than nylon 6 such as nylon-6,6, nylon-4,10; cellulose derivatives such as nitrocellulose, acetyl cellulose, butyric-acetyl cellulose;

collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof or a porous carbon material such as an activated carbon. Further, a number of the absorptive regions 4 of the biochemical analysis unit 1 can be formed of any of various inorganic porous materials including, for example, metals such as platinum, gold, iron, silver, nickel, aluminum and the like; metal oxides such as alumina, silica, titania, zeolite and the like; metal salts such as hydroxy apatite, calcium sulfate and the like; and composite materials thereof or a bundle of a plurality of fibers.

Moreover, in the above-described embodiment, although the biochemical analysis unit 1 includes the substrate 2 made of aluminum, it is not absolutely necessary to make the substrate 2 of the biochemical analysis unit 1 of aluminum but the substrate 2 of the biochemical analysis unit 1 may be made of other kinds of material. It is preferable to make the substrate 2 of the biochemical analysis unit 1 of a material capable of attenuating light energy and/or radiation energy but a material for forming the substrate 2 of the biochemical analysis unit 1 is not particularly limited. The substrate 2 of the biochemical analysis unit 1 can be formed of either an inorganic compound material or an organic compound material and is preferably formed of a metal material, a ceramic material or a plastic material. Illustrative examples of inorganic compound materials usable for forming the substrate 2 of the biochemical analysis unit 1 include metals such as gold, silver, copper, zinc, aluminum, titanium, tantalum, chromium, steel, nickel, cobalt, lead, tin, selenium and the like; alloys such as brass, stainless, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like. High molecular compounds are preferably used as an organic compound material for forming the substrate 2 of the biochemical analysis unit 1 and illustrative examples thereof include polyolefins such as polyethylene, polypropylene and the like; acrylic resins such as polymethyl methacrylate, polybutylacrylate/ polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4, 10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Further, in the above-described embodiment, although a number of the absorptive regions 4 of the biochemical analysis unit 1 are formed by charging nylon-6 in a number of the through-holes 3 formed in the substrate 2 made of aluminum, it is possible to form a number of recesses in the substrate 2 made of aluminum instead of the through-hole 3 so as to be spaced apart from each other and charging nylon-6 in a number of the recesses, thereby forming a number of the absorptive regions 4.

Furthermore, in the above-described embodiment, although a number of the absorptive regions 4 of the biochemical analysis unit 1 are formed by charging nylon-6 in a number of the through-holes 3 formed in the substrate 2 made of aluminum, a number of the absorptive regions 4 may be formed by pressing an absorptive membrane made of an absorptive material such as nylon-6 into a number of the through-holes 3 formed in the substrate 2 of the biochemical analysis unit 1.

Moreover, in the above-described embodiment, although a number of the absorptive regions 4 of the biochemical analysis unit 1 are formed by charging nylon-6 in a number of the through-holes 3 formed in the substrate 2 made of aluminum, a number of absorptive regions may be formed by closely contacting a perforated plate formed with a number of through-holes onto one surface of an absorptive substrate and spotting a solution containing specific binding substances in the absorptive substrate within a number of the through-holes.

Further, in the above-described embodiment, biochemical analysis data are produced by photoelectrically detecting or chemiluminescence emission 18 using the cooled CCD area sensor 15, biochemical analysis data can be produced by photoelectrically detecting or chemiluminescence emission 18 using a CCD area sensor provided with no cooling means and instead of the CCD area sensor, another type of solid state sensor such as a CID (charge injection device), a PDA (photodiode array), a MOS type imaging device and the like may be used.

According to the present invention, it is possible to provide a method for producing biochemical analysis data and an apparatus used therefor which can detect chemiluminescence emission released from a specimen region containing a small amount of a labeling substance which generates chemiluminescence emission when it contacts a chemiluminescent substrate as well as chemiluminescence emission released from a specimen region containing a large amount of the labeling substance in a desired manner, thereby producing biochemical analysis data having an excellent quantitative characteristic.

## Claims

1. A method for producing biochemical analysis data comprising steps of (i) selectively binding a substance derived from a living organism and labeled with a labeling substance which generates chemiluminescence emission when it contacts a chemiluminescent substrate with specific binding substances whose structure and characteristics are known and which are contained in a plurality of absorptive regions formed in a biochemical analysis unit so as to be spaced apart from each other, or selectively binding a substance derived from a living organism and labeled with a hapten with the specific binding substances whose structure and characteristics are known and which are contained in the plurality of absorptive regions formed in the biochemical analysis unit so as to be spaced apart from each other and binding an antibody for the hapten labeled with an enzyme having a property to generate chemiluminescence emission when it contacts a chemiluminescent substrate with the hapten by an antigen-antibody reaction, (ii) bringing the labeling substance selectively contained in the plurality of absorptive regions of the biochemical analysis unit into contact with a chemiluminescent substrate, thereby causing it to release chemiluminescence emission, (iii) photoelectrically detecting the chemiluminescence emission released from the plurality of absorptive regions of the biochemical analysis unit for a first exposure time period using a solid state area sensor to produce an analog signal for each of the plurality of absorptive regions of the biochemical analysis unit, (iv) digitizing the analog signal to produce a digital signal for each of the plurality of absorptive regions of the biochemical analysis unit and (v) storing the digital signals in a memory,**characterised in that** the method further encompasses the steps of; (vi) photoelectrically detecting the chemiluminescence emission released from the plurality of absorptive regions of the biochemical analysis unit for a second exposure time period longer than the first exposure time period to produce an analog signal for each of the plurality of absorptive regions of the biochemical analysis unit, (vii) digitizing the analog signal to produce a digital signal for each of the plurality of absorptive regions of the biochemical analysis unit, (viii) storing the digital signals in the memory, (ix) comparing a signal intensity of the digital signal produced by photoelectrically detecting the chemiluminescence emission released from each of the absorptive regions of the biochemical analysis unit for the second exposure time period and stored in the memory with a saturated value of the signal intensity, (x) determining a signal intensity of a digital signal which is lower than the saturated value as biochemical analysis data of the corresponding absorptive region of the biochemical analysis unit to store it in the memory, (xi) and adopting a signal intensity of a digital signal which is generated by photoelectrically detecting the chemiluminescence emission released from an absorptive region of the biochemical analysis unit for the first exposure time period and stored in the memory when a signal intensity of a digital signal generated by photoelectrically detecting the chemiluminescence emission released from the absorptive region of the biochemical analysis unit for the second exposure time period is equal to or higher than the saturated value, (xii) thereby producing biochemical analysis data.

2. A Method of producing biochemical analysis data in accordance with claim 1 which further comprises steps of producing a correction coefficient (K) based on a ratio of a signal intensity (SC(2)) of a digital signal generated by phosphoelectrically detecting chemiluminescence emission released form an absorptive region C of the biochemical analysis unit for the second exposure time (T2) and stored in the memory and whose signal intensity is lower than the saturated value and a signal (SC(1)) of a digital signal generated by photoelectrically detecting chemiluminescence emission released from the absorptive region C for the first exposure time period (T1) and stored in the memory, and multiplying a signal intensity (SD(1)) of the digital signal generated by photoelectrically detecting chemiluminescence emission released from an absorptive region D of the biochemical analysis unit for the first exposure time period (T1) and stored in the memory by the correction coefficient (K) when the signal intensity (SD(2)) of the digital signal generated by photoelectrically detecting chemoluminescence emission released from the absorptive region D for the second exposure time (T2) is equal to or higher than the saturated value, thereby producing biochemical analysis data of the absorptive region.

3. A method of producing biochemical analysis data in accordance with Claim 2 which further comprises the step of dividing a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient.

4. A method of producing biochemical analysis data in accordance with any one of Claims 1 to 3 wherein biochemical analysis data are produced by leading chemiluminescence emission released from the plurality of absorptive regions of the biochemical analysis unit by a plurality of light guide members disposed in such a manner that each of the plurality of absorptive regions faces one of the light collecting end portions of the plurality of light guide members to a light detector and photoelectrically detecting chemiluminescence emission by the light detector.

5. A method of producing biochemical analysis data in accordance with Claim 4 wherein each of the plurality of light guide members is formed of at least one optical fiber.

6. A method of producing biochemical analysis data in accordance with Claim 4 wherein each of the plurality of light guide members is formed of an optical fiber bundle constituted by a plurality of optical fibers.

7. A method of producing biochemical analysis data in accordance with any one of Claims 4 to 6 wherein the plurality of light guide members are gathered in the vicinity of end portions opposite to the light collecting end portions.

8. A method of producing biochemical analysis data in accordance with any one of Claims 4 to 7 wherein the plurality of light guide members are mounted on a fixing head in the vicinity of the light collecting end portions so that each of the light collecting end portions of the plurality of light guide members is disposed to face one of the plurality of absorptive regions.

9. A method of producing biochemical analysis data in accordance with any one of Claims 1 to 8 wherein the solid state area sensor is constituted by a cooled CCD area sensor.

10. A method of producing biochemical analysis data in accordance with any one of Claims 1 to 9 wherein the biochemical analysis unit includes a substrate formed with a plurality of holes to be spaced apart from each other and the plurality of absorptive regions are formed by charging an absorptive material in the plurality of the holes formed in the substrate.

11. A method of producing biochemical analysis data in accordance with of Claim 10 wherein the substrate of the biochemical analysis unit has a property of attenuating light energy.

12. A method of producing biochemical analysis data in accordance with Claim 11 wherein the substrate of the biochemical analysis unit has a property of reducing the energy of light to 1/5 or less when the light travels in the substrate by a distance equal to that between neighboring absorptive regions.

13. A method of producing biochemical analysis data in accordance with Claim 11 or 12 wherein the substrate of the biochemical analysis unit is made of a material selecting from a group consisting of a metal material, a ceramic material and a plastic material.

14. A method of producing biochemical analysis data in accordance with any one of Claims 1 to 13 wherein the biochemical analysis unit is formed with 10 or more absorptive regions.

15. A method of producing biochemical analysis data in accordance with any one of Claims 1 to 14 wherein each of the plurality of absorptive regions is formed in the biochemical analysis unit to have a size of less than 5 mm$^2$.

16. A method of producing biochemical analysis data in accordance with any one of Claims 1 to 15 wherein the plurality of absorptive regions are formed in the biochemical analysis unit at a density of 10 or more per cm$^2$.

17. An apparatus for producing biochemical analysis data comprising (i) a sample stage for placing a biochemical analysis unit in which a plurality of absorptive regions are formed so as to be spaced apart from each other and (ii) a substance derived from a living organism and labeled with a labeling substance which generates chemiluminescence emission when it contacts a chemiluminescent substrate is selectively bound with specific binding substances whose structure or characteristics are known contained in the plurality of absorptive regions, (iii) a solid state area sensor for photoelectrically detecting chemiluminescence emission released from the plurality of absorptive regions of the biochemical analysis unit and generating an analog signal for each of the plurality of absorptive regions of the biochemical analysis unit, (iv) a plurality of light guide members disposed in such a manner that each of light collecting end portions of the light guide members faces one of the plurality of absorptive regions of the biochemical analysis unit placed on the sample stage and adapted for leading chemiluminescence emission released from the plurality of absorptive regions of the biochemical analysis unit to the solid state area sensor, (v) an A/D converter for digitizing analog signals generated by the solid state area sensor to produce a digital signal for each of the plurality of absorptive regions of the biochemical analysis unit, (vi) a memory for storing the digital signal generated by the A/D converter for each of the plurality of absorptive regions of the biochemical analysis unit, (vii) data processing means for producing biochemical analysis data for each of the plurality of absorptive regions of the biochemical analysis unit based on the digital signal of each of the plurality of absorptive regions of the biochemical analysis unit, and (viii) a control means for controlling the solid state area sensor, the A/D converter and the data processing means, the control means being adapted for (a) controlling the solid state area sensor so as to photoelectrically detect chemiluminescence emission released from the absorptive regions of the biochemical analysis unit for a first exposure time period, thereby generating an analog signal for each of the absorptive regions of the biochemical analysis unit and to photoelectrically detect chemiluminescence emission released from the absorptive regions of the biochemical analysis unit for a second exposure time period longer than the first exposure time period, thereby generating an analog signal for each of the absorptive regions of the biochemical analysis unit, (b) controlling the A/D converter so as to digitize the analog signal of each of the absorptive regions of the biochemical analysis unit generated by the solid state area sensor and to store it in the memory, and (c) controlling the data processing means to compare a signal intensity of the digital signal of each of the absorptive regions of the biochemical analysis unit generated by photoelectrically detecting chemiluminescence emission released from the absorptive regions of the biochemical analysis unit for the second exposure time period and stored in the memory with a saturated value of the signal intensity, to determine the signal intensity of the digital signal which is lower than the saturated value as biochemical analysis data of the absorptive region of the biochemical analysis unit, to store them in the memory, and (d) to adopt a signal intensity of a digital signal which is generated by photoelectrically detecting the chemiluminescence emission released from an absorptive region of the biochemical analysis unit for the first exposure time period and stored in the memory when a signal intensity of a digital signal generated by photoelectrically detecting the chemiluminescence emission released from the absorptive region of the biochemical analysis unit for the second exposure time period is equal to or higher than the saturated value, (e) thereby producing biochemical analysis data of the plurality of the absorptive regions of the biochemical analysis unit, **characterized in that** the data processing means is constituted so as to further produce a correction coefficient (K) based on a ratio of a signal intensity (SC(2)) of a digital signal generated by photoelectrically detecting chemiluminescence emission released from an absorptive region C of the biochemical analysis unit for the second exposure time period (T2) and stored in the memory and whose signal intensity is lower than the saturated value and a signal intensity (SC (1)) of a digital signal generated by photoelectrically detecting chemiluminescence emission released from the absorptive region C of the biochemical analysis unit for the first exposure time period (T1) and stored in the memory, and to multiply a signal intensity (SD (1)) of the digital signal generated by photoelectrically detecting chemiluminescence emission released from an absorptive region D of the biochemical analysis unit for the first exposure time period (T1) and stored in the memory by the correction coefficient (K) when the signal intensity (SD (2)) of the digital signal generated by photo electrically detecting chemiluminescence emission released from the absorptive region D for the second exposure time (T2) is equal to or higher than the saturated value thereby producing biochemical analysis data of the absorptive region.

18. An apparatus for producing biochemical analysis data in accordance with Claim 17 wherein the data processing means is constituted so as to divide a signal intensity of a digital signal whose signal intensity is maximum among signal intensities of digital signals generated by photoelectrically detecting chemiluminescence emission released from the absorptive regions of the biochemical analysis unit for the second exposure time period and stored in the memory and whose signal intensity is lower than the saturated value by a signal intensity of a digital signal produced by photoelectrically detecting chemiluminescence emission released from the absorptive region of the biochemical analysis unit for the first exposure time period and stored in the memory, thereby producing the correction coefficient.

19. An apparatus for producing biochemical analysis data in accordance with any one of Claims 17 and 18 wherein each of the plurality of light guide members is formed of at least one optical fiber.

20. An apparatus for producing biochemical analysis data in accordance with any one of Claims 17 and 18 wherein each of the plurality of light guide members is formed of an optical fiber bundle constituted by a plurality of optical fibers.

21. An apparatus for producing biochemical analysis data in accordance with any one of Claims 17 to 20 wherein the plurality of light guide members are gathered in the vicinity of end portions opposite to the light collecting end portions.

22. An apparatus for producing biochemical analysis data in accordance with any one of Claims 17 to 21 wherein the plurality of light guide members are mounted on a fixing head in the vicinity of the light collecting end portions so that each of the light collecting end portions of the plurality of light guide members is disposed to face one of the plurality of absorptive regions.

23. An apparatus for producing biochemical analysis data in accordance with any one of Claims 17 to 22 wherein the solid state area sensor is constituted by a cooled CCD area sensor.

**Patentansprüche**

1. Verfahren zum Erzeugen von biochemischen Analysedaten, umfassend die Schritte: (i) selektives Binden einer Substanz, die von einem lebenden Organismus abgeleitet wurde und mit einer Markierungssubstanz markiert wurde, die Chemilumineszenz-Emission zeigt, wenn sie ein Chemilumineszenzsubstrat kontaktiert, mit spezifischen Bindesubstanzen, deren Struktur und Charakteristika bekannt sind, und die in einer Mehrzahl von absorptiven Zonen enthalten sind, die in einer biochemischen Analyseeinheit getrennt voneinander ausgebildet sind, oder selektives Binden einer Substanz, die von einem lebenden Organismus abgeleitet wurde und mit einem Hapten markiert wurde, mit spezifischen Bindesubstanzen, deren Struktur und Charakteristika bekannt sind, und die in den mehreren absorptiven Zonen enthalten sind, die in der biochemischen Analyseeinheit beabstandet voneinander ausgebildet sind, und Binden eines Antikörpers für das Hapten, welches markiert ist mit einem Enzym, welches die Eigenschaft besitzt, Chemilumineszenz-Emission zu zeigen, wenn es ein chemilumineszentes Substrat mit dem Hapten kontaktiert bei einer Antigen-Antikörper-Reaktion, (ii) Bringen der Markierungssubstanz, die selektiv in den mehreren absorptiven Zonen der biochemischen Analyseeinheit enthalten ist, in Berührung mit einem chemilumineszenten Substrat, um dadurch die Freigabe einer Chemilumineszenz-Emission zu veranlassen, (iii) photoelektrisches Detektieren der von den mehreren absorptiven Zonen der biochemischen Analyseeinheit freigesetzten Chemilumineszenz-Emission für eine erste Belichtungszeitspanne unter Verwendung eines Festkörper-Flächensensors, um ein Analogsignal für jede der mehreren absorptiven Zonen der biochemischen Analyseeinheit zu erzeugen, (iv) Digitalisieren des Analogsignals, um ein digitales Signal für jede der mehreren absorptiven Zonen der biochemischen Analyseeinheit zu erzeugen, und (v) Speichern der digitalen Signale in einem Speicher, **dadurch gekennzeichnet, dass** das Verfahren außerdem folgende Schritte aufweist: (vi) photoelektrisches Detektieren der von den mehreren absorptiven Zonen der biochemischen Analyseeinheit freigesetzten Chemilumineszenz-Emission für eine zweite Belichtungszeitspanne, die länger ist als die erste Belichtungszeitspanne, um ein Analogsignal für jede der mehreren absorptiven Zonen der biochemischen Analyseeinheit zu erzeugen, (vii) Digitalisieren des Analogsignals, um ein digitales Signal für jede der mehreren absorptiven Zonen der biochemischen Analyseeinheit zu erzeugen, (viii) Speichern der digitalen Signale in dem Speicher, (ix) Vergleichen einer Signalintensität des digitalen Signals, produziert durch das photoelektrische Detektieren der Chemilumineszenz-Emission, die von jeder der absorptiven Zonen der biochemischen Analyseeinheit freigesetzt wurde, für die zweite Belichtungszeitspanne, und abgespeichert in dem Speicher, mit einem gesättigten Wert der Signalintensität, (x) Bestimmen einer Signalintensität eines digitalen Signals, welches kleiner ist als der gesättigte Wert, als biochemische Analysedaten der entsprechenden absorptiven Zone der biochemischen Analyseeinheit, um den Wert in dem Speicher zu speichern, und (xi) Verwenden einer Signalintensität eines digitalen Signals, welches erzeugt wurde durch photoelektrisches Detektieren der Chemilumineszenz-Emission, die von einer absorptiven Zone der biochemischen Analyseeinheit für die erste Belichtungszeitspanne freigesetzt und in dem Speicher gespeichert wurde, wenn eine Signalintensität eines digitalen Signals, die erzeugt wurde durch photoelektrisches Detektieren der Chemilumineszenz-Emission, die von der absorptiven Zone der biochemischen Analyseeinheit für die zweite Belichtungszeitspanne freigesetzt wurde, gleich oder größer ist als der gesättigte Wert, (xii) um dadurch biochemische Analysedaten zu erzeugen.

2. Verfahren nach Anspruch 1, weiterhin umfassend die Schritte des Erzeugens eines Korrekturkoeffizienten (K) basierend auf einem Verhältnis einer Signalintensität (SC(2)) eines digitalen Signals, welches erzeugt wird durch photoelektrisches Detektieren der Chemilumineszenz-Emission, die von einer absorptiven Zone C der biochemischen Analyseeinheit für die zweite Belichtungszeit (T2) freigesetzt wurde und in dem Speicher gespeichert wurde, und dessen Signalintensität geringer ist als der gesättigte Wert, und eines Signals (SC(1)) eines digitalen Signals, welches erzeugt wurde durch photoelektrisches Detektieren der Chemilumineszenz-Emission, freigesetzt von der absorptiven Zone C in der ersten Belichtungszeitspanne (T1) und abgespeichert in dem Speicher, und des Multiplizierens einer Signalintensität (SD(1)) des digitalen Signals, welches erzeugt wurde durch photoelektrisches Detektieren der Chemilumineszenz-Emission, die von einer absorptiven Zone D der biochemischen Analyseeinheit für die erste Belichtungszeitspanne (T1) freigesetzt und in dem Speicher abgespeichert wurde, mit dem Korrekturkoeffizienten (K), wenn die Signalintensität (SD(2)) des digitalen Signals, welches durch photoelektrisches Detektieren der Chemilumineszenz-Emission aus der absorptiven Zone D für die zweite Belichtungszeit (T2) erzeugt wurde, gleich oder größer ist als der gesättigte Wert, um dadurch biochemische Analysedaten für die absorptive Zone zu erzeugen.

3. Verfahren nach Anspruch 2, weiterhin umfassend den Schritt des Dividierens einer Signalintensität eines digitalen Signals, die unter den Signalintensitäten von digitalen Signalen einen Maximumwert hat, welche durch photoelektrisches Detektieren der Chemilumineszenz-Emission erzeugt wurden, die von den absorptiven Zonen der biochemischen Analyseeinheit für die zweite Belichtungszeitspanne freigesetzt und in dem Speicher abgespeichert wurde, und dessen Signalintensität kleiner ist als der gesättigte Wert, durch eine Signalintensität eines digitalen Signals, welches erzeugt wurde durch photoelektrisches Detektieren der Chemilumineszenz-Emission, die von der absorptiven Zone der biochemischen Analyseeinheit für die erste Belichtungszeitspanne freigesetzt und in dem Speicher gespeichert wurde, um dadurch den Korrekturkoeffizienten zu bilden.

4. Verfahren nach einem der Ansprüche 1 bis 3, bei dem biochemische Analysedaten dadurch erstellt werden, dass Chemilumineszenz-Emission, die von mehreren absorptiven Zonen der biochemischen Analyseeinheit freigesetzt wurde, von mehreren Lichtleitelementen, die so angeordnet sind, dass jede der mehreren absorptiven Zonen einem der Lichtsammel-Endbereiche der mehreren Lichtleitelemente gegenüberliegen, zu einem Lichtdetektor geführt und die Chemilumineszenz-Emission von dem Lichtdetektor photoelektrisch aufgenommen wird.

5. Verfahren nach Anspruch 4, bei dem jedes der mehreren Lichtleitelemente durch mindestens eine optische Faser gebildet wird.

6. Verfahren nach Anspruch 4, bei dem jedes der mehreren Lichtleitelemente gebildet wird durch ein optisches Faserbündel, bestehend aus einer Mehrzahl optischer Fasern.

7. Verfahren nach einem der Ansprüche 4 bis 6, bei dem die mehreren Lichtleitelemente in der Nähe der den Lichtsammel-Endbereichen gegenüberliegenden Endbereiche gesammelt werden.

8. Verfahren nach einem der Ansprüche 4 bis 7, bei dem die mehreren Lichtleitelemente an einem Fixierkopf in der Nähe der Lichtsammel-Endbereiche gelagert sind, so dass die Lichtsammel-Endbereiche der mehreren Lichtleitelemente so angeordnet sind, dass sie den mehreren absorptiven Zonen gegenüberliegen.

9. Verfahren nach einem der Ansprüche 1 bis 8, bei dem der Festkörper-Flächensensor durch einen gekühlten CCD-Flächensensor gebildet wird.

10. Verfahren nach einem der Ansprüche 1 bis 9, bei dem die biochemische Analyseeinheit ein Substrat enthält, ausgebildet mit einer Mehrzahl von Löchern im gegenseitigen Abstand, wobei die mehreren absorptiven Zonen gebildet werden durch Einbringen eines absorptiven Materials in die in dem Substrat gebildeten mehreren Löcher.

11. Verfahren nach Anspruch 10, bei dem das Substrat der biochemischen Analyseeinheit die Eigenschaft besitzt, Lichtenergie zu dämpfen.

12. Verfahren nach Anspruch 11, bei dem das Substrat der biochemischen Analyseeinheit die Eigenschaft hat, die Lichtenergie auf 1/5 oder weniger zu reduzieren, wenn das Licht in dem Substrat eine Strecke zurücklegt, die gleich ist derjenigen zwischen benachbarten absorptiven Zonen.

13. Verfahren nach Anspruch 11 oder 12, bei dem das Substrat der biochemischen Analyseeinheit aus einem Material besteht, welches ausgewählt ist aus einer Gruppe, die aus einem Metallwerkstoff, einem keramischen Werkstoff und einem Kunststoff besteht.

14. Verfahren nach einem der Ansprüche 1 bis 13, bei dem die biochemische Analyseeinheit durch 10 oder mehr absorptive Zonen gebildet ist.

15. Verfahren nach einem der Ansprüche 1 bis 14, bei dem jede der mehreren absorptiven Zonen in der biochemischen Analyseeinheit mit einer Größe von weniger als 5 mm$^2$ gebildet ist.

16. Verfahren nach einem der Ansprüche 1 bis 15, bei dem die mehreren absorptiven Zonen in der biochemischen Analyseeinheit mit einer Dichte von 10 oder mehr pro cm$^2$ gebildet sind.

17. Vorrichtung zum Erzeugen von biochemischen Analysedaten, umfassend: (i) eine Probenbühne zum Platzieren einer biochemischen Analyseeinheit, in der mehrere absorptive Zonen mit Abstand voneinander gebildet sind, und (ii) eine Substanz, die von einem lebenden Organismus abgeleitet ist und mit einer Markierungssubstanz markiert ist, die Chemilumineszenz-Emission erzeugt, wenn sie ein chemilumineszentes Substrat kontaktiert, selektiv gebunden wird mit spezifischen Bindesubstanzen, deren Struktur oder Charakteristik bekannt ist, enthalten in den mehreren absorptiven Zonen, (iii) einen Festkörper-Flächensensor zum photoelektrischen Detektieren von Chemilumineszenz-Emission, die von den mehreren absorptiven Zonen der biochemischen Analyseeinheit freigesetzt wurde, und zum Erzeugen eines Analogsignals für jede der mehreren absorptiven Zonen der biochemischen Analyseeinheit, (iv) mehrere Lichtleitelemente, die so angeordnet sind, dass jeder Lichtsammel-Endbereich der Lichtleichtelemente einer der mehreren absorptiven Zonen der biochemischen Analyseeinheit auf der Probenbühne gegenüberliegt, und ausgebildet zu dem Zweck, Chemilumineszenz-Emission, die von den mehreren absorptiven Zonen der biochemischen Analyseeinheit freigesetzt wurde, zu dem Festkörper-Flächensensor zu leiten, (v) einen A/D-Wandler zum Digitalisieren von Analogsignalen, die von dem Festkörper-Flächensensor erzeugt wurden, um ein digitales Signal für jede der absorptiven Zonen der biochemischen Analyseeinheit zu erzeugen, (vi) einen Speicher zum Speichern des digitalen Signals, welches von dem A/D-Wandler für jede der mehreren absorptiven Zonen der biochemischen Analyseeinheit erzeugt wurde, (vii) eine Datenverarbeitungseinrichtung zum Erzeugen biochemischer Analysedaten für jede der mehreren absorptiven Zonen der biochemischen Analyseeinheit, basierend auf dem digitalen Signal jeder der mehreren absorptiven Zonen der biochemischen Analyseeinheit, und (viii) eine Steuereinrichtung zum Steuern des Festkörper-Flächensensors, des A/D-Wandlers und der Datenverarbeitungseinrichtung, wobei die Steuereinrichtung dazu ausgebildet ist, (a) den Festkörper-Flächensensor derart zu steuern, dass er Chemilumineszenz-Emission, die von den absorptiven Zonen der biochemischen Analyseeinheit während einer ersten Belichtungszeitspanne freigesetzt wurde, photoelektrisch detektiert, um dadurch ein Analogsignal für jede der absorptiven Zonen der biochemischen Analyseeinheit zu erzeugen, und um photoelektrisch Chemilumineszenz-Emission zu detektieren, die von den absorptiven Zonen der biochemischen Analyseeinheit während einer zweiten Belichtungszeitspanne, die länger als die erste Belichtungszeitspanne ist, freigesetzt wird, um dadurch ein Analogsignal für jede der absorptiven Zonen der biochemischen Analyseeinheit zu erzeugen, (b) den A/D-Wandler so zu steuern, dass er das Analogsignal für jede der absorptiven Zonen der biochemischen Analyseeinheit, das von dem Festkörper-Flächensensor erzeugt wurde, digitalisiert und den Wert in dem Speicher speichert, und (c) die Datenverarbeitungseinrichtung so zu steuern, dass sie eine Signalintensität des digitalen Signals für jede der absorptiven Zonen der biochemischen Analyseeinheit, das durch photoelektrisches Detektieren von Chemilumineszenz-Emission erzeugt wurde, die von den absorptiven Zonen der biochemischen Analyseeinheit für die zweite Belichtungszeitspanne erzeugt und in dem Speicher abgespeichert wurde, vergleicht mit einem gesättigten Wert der Signalintensität, um die Signalintensität des digitalen Signals, die geringer als der gesättigte Wert ist, als biochemische Analysedaten für die absorptive Zone der biochemischen Analyseeinheit zu bestimmen und sie in dem Speicher abzuspeichern, und (d) eine Signalintensität eines digitalen Signals, das durch photoelektrisches Detektieren der Chemilumineszenz-Emission erzeugt wurde, die von einer absorptiven Zone der biochemischen Analyseeinheit während der ersten Belichtungszeitspanne freigesetzt wurde, und das in dem Speicher gespeichert wird, wenn eine Signalintensität eines digitalen Signals, welches durch photoelektrisches Detektieren der Chemilumineszenz-Emission erzeugt wird, die von der absorptiven Zone der biochemischen Analyseeinheit für die zweite Belichtungszeitspanne freigesetzt wurde, gleich oder größer ist als der gesättigte Wert, (e) um dadurch biochemische Analysedaten der mehreren absorptiven Zonen der biochemischen Analyseeinheit zu erzeugen, **dadurch gekennzeichnet, dass** die Datenverarbeitungseinrichtung dazu ausgebildet ist, außerdem einen Korrekturkoeffizienten (K) zu erzeugen, basierend auf einem Verhältnis einer Signalintensität (SC(2)) eines digitalen Signals, erzeugt durch photoelektrisches Detektieren von Chemilumineszenz-Emission, die von einer absorptiven Zone C der biochemischen Analyseeinheit während der zweiten Belichtungszeitspanne (T2) freigesetzt wurde und in dem Speicher gespeichert wurde, und dessen Signalintensität kleiner als der gesättigte Wert ist, und einer Signalintensität (SC(1)) eines digitalen Signals, erzeugt durch photoelektrisches Detektieren der Chemilumineszenz-Emission, die von der absorptiven Zone C der biochemischen Analyseeinheit für die erste Belichtungszeitspanne (T1) freigesetzt und in dem Speicher gespeichert wurde, und um eine Signalintensität (SD(1)) des digitalen Signals, erzeugt durch photoelektrisches Detektieren der Chemilumineszenz-Emission, die von einer absorptiven Zone D der biochemischen Analyseeinheit während der ersten Belichtungszeitspanne (T1) freigesetzt und in dem Speicher gespeichert wurde, zu multiplizieren mit dem Korrekturkoeffizienten (K), wenn die Signalintensität (SD(2)) des digitalen Signals, welches durch photoelektrisches Detektieren der Chemilumineszenz-Emission erzeugt wurde, die von der absorptiven Zone D während der zweiten Belichtungszeitspanne (T2) freigesetzt wurde, gleich oder größer ist als der gesättigte Wert, um dadurch biochemische Analysedaten für die absorptive Zone zu erzeugen.

18. Vorrichtung nach Anspruch 17, bei der die Datenverarbeitungseinrichtung so ausgebildet ist, dass sie eine Signalintensität eines digitalen Signals, dessen Signalintensität unter den Signalintensitäten von Signalen ein Maximum ist, die durch photoelektrisches Detektieren der Chemilumineszenz-Emission aus den absorptiven Zonen der biochemischen Analyseeinheit während der zweiten Belichtungszeitspanne erzeugt und in dem Speicher abgespeichert wurden, und deren Signalintensität geringer ist als der gesättigte Wert, dividiert durch eine Signalintensität eines digitalen Signals, welches durch photoelektrisches Detektieren der Chemilumineszenz-Emission erzeugt wird, die von der absorptiven Zone der biochemischen Analyseeinheit während der ersten Belichtungszeitspanne freigesetzt und in dem Speicher gespeichert wurde, um dadurch den Korrekturkoeffizienten zu erzeugen.

19. Vorrichtung nach Anspruch 17 und 18, bei der jedes der mehreren Lichtleitelemente durch mindestens eine optische Faser gebildet wird.

20. Vorrichtung nach einem der Ansprüche 17 und 18, bei der die mehreren Lichtleitelemente durch ein optisches Faserbündel aus mehreren optischen Fasern gebildet wird.

21. Vorrichtung nach einem der Ansprüche 17 bis 20, bei der die mehreren Lichtleitelemente in der Nähe der Endbereiche gegenüber den Lichtsammel-Endbereichen zusammengefasst sind.

22. Vorrichtung nach einem der Ansprüche 17 bis 21, bei der die mehreren Lichtleitelemente an einem Fixierkopf in der Nähe der Lichtsammel-Endbereiche derart gelagert sind, dass jeder der Lichtsammel-Endbereiche der mehreren Lichtleitelemente sich gegenüber einer der mehreren absorptiven Zonen befinden.

23. Vorrichtung nach einem der Ansprüche 17 bis 22, bei der der Festkörper-Flächensensor gebildet wird durch einen gekühlten CCD-Flächensensor.

## Revendications

1. Procédé de production de données d'analyse biochimique comprenant les étapes consistant à (i) lier sélectivement une substance dérivée d'un organisme vivant et marquée avec une substance marquante qui génère une émission de chimioluminescence lorsqu'elle vient en contact avec un substrat chimioluminescent avec des substances de liaison spécifiques dont la structure et les caractéristiques sont connues et qui sont contenues dans une pluralité de régions absorbantes formées dans une unité d'analyse biochimique de façon à être espacées les unes des autres, ou lier sélectivement une substance dérivée d'un organisme vivant et marquée avec un haptène avec les substances de liaison spécifiques dont la structure et les caractéristiques sont connues et qui sont contenues dans la pluralité de régions absorbantes formées dans l'unité d'analyse biochimique de façon à être espacées les unes des autres et lier un anticorps pour l'haptène marqué avec une enzyme ayant la propriété de générer une émission de chimioluminescence lorsqu'elle vient en contact avec un substrat chimioluminescent avec l'haptène par une réaction antigène-anticorps, (ii) amener la substance marquante sélectivement contenue dans la pluralité de régions absorbantes de l'unité d'analyse biochimique au contact avec un substrat chimioluminescent, l'amenant ainsi à libérer une émission de chimioluminescence, (iii) détecter photoélectriquement l'émission de chimioluminescence libérée de la pluralité de régions absorbantes de l'unité d'analyse biochimique pendant une première période d'exposition en utilisant un capteur de zone à semi-conducteur pour produire un signal analogique pour chacune de la pluralité de régions absorbantes de l'unité d'analyse biochimique, (iv) numériser le signal analogique pour produire un signal numérique pour chacune de la pluralité de régions absorbantes de l'unité d'analyse biochimique, et (v) stocker les signaux numériques dans une mémoire, **caractérisé en ce que** le procédé comprend en outre les étapes consistant à (vi) détecter photoélectriquement l'émission de chimioluminescence libérée à partir de la pluralité de régions absorbantes de l'unité d'analyse biochimique pendant une seconde période d'exposition plus longue que la première période d'exposition pour produire un signal analogique pour chacune de la pluralité de régions absorbantes de l'unité d'analyse biochimique, (vii) numériser le signal analogique pour produire un signal numérique pour chacune de la pluralité de régions absorbantes de l'unité d'analyse biochimique, (viii) stocker les signaux numériques dans la mémoire, (ix) comparer une intensité de signal du signal numérique produit en détectant photoélectriquement l'émission de chimioluminescence libérée à partir de chacune des régions absorbantes de l'unité d'analyse biochimique pendant la seconde période d'exposition et stockée dans la mémoire avec une valeur saturée de l'intensité de signal, (x) déterminer une intensité de signal d'un signal numérique qui est plus basse que la valeur saturée comme données d'analyse biochimique de la région absorbante correspondante de l'unité d'analyse biochimique pour la stocker dans la mémoire, (xi) et adopter une intensité de signal d'un signal numérique qui est généré en détectant photoélectriquement l'émission de chimioluminescence libérée d'une région absorbante de l'unité d'analyse biochimique pendant la première période d'exposition et stockée dans la mémoire lorsqu'une intensité de signal d'un signal numérique généré en détectant photoélectriquement l'émission de chimioluminescence libérée à partir de la région absorbante de l'unité d'analyse biochimique pendant la seconde période d'exposition est égale ou supérieure à la valeur saturée, (xii) produire ainsi des données d'analyse biochimique.

2. Procédé de production de données d'analyse biochimique selon la revendication 1, qui comprend en outre les étapes consistant à produire un coefficient de correction (K) basé sur un rapport d'intensité de signal (SC(2)) d'un signal numérique généré en détectant photoélectriquement une émission de chimioluminescence libérée à partir d'une région absorbante C de l'unité d'analyse biochimique pendant la seconde période d'exposition (T2) et stockée dans la mémoire et dont l'intensité de signal est plus basse que la valeur saturée et un signal (SC(1)) d'un signal numérique généré en détectant photoélectriquement une émission de chimioluminescence libérée à partir de la région absorbante C pendant la première période d'exposition (T1) et stockée dans la mémoire, et multiplier une intensité de signal (SD (1)) du signal numérique généré en détectant photoélectriquement une émission de chimioluminescence libérée à partir d'une région absorbante D de l'unité d'analyse biochimique pendant la première période d'exposition (T1) et stockée dans la mémoire par le coefficient de correction (K) lorsque l'intensité de signal (SD(2)) du signal numérique généré en détectant photoélectriquement l'émission de chimioluminescence libérée à partir de la région absorbante D pendant la seconde période d'exposition (T2) est égale à ou supérieure à la valeur saturée, produisant ainsi des données d'analyse biochimique de la région absorbante.

3. Procédé de production de données d'analyse biochimique selon la revendication 2, qui comprend en outre l'étape consistant à diviser une intensité de signal d'un signal numérique dont l'intensité de signal est maximale parmi des intensités de signal de signaux numériques générés en détectant photoélectriquement une émission de chimioluminescence libérée à partir des régions absorbantes de l'unité d'analyse biochimique pendant la seconde période d'exposition et stockée dans la mémoire et dont l'intensité de signal est plus basse que la valeur saturée par une intensité de signal d'un signal numérique produit en détectant photoélectriquement une émission de chimioluminescence libérée à partir de la région absorbante de l'unité d'analyse biochimique pendant la première période d'exposition et stockée dans la mémoire, produisant ainsi le coefficient de correction.

4. Procédé de production de données d'analyse biochimique selon l'une quelconque des revendications 1 à 3, dans lequel les données d'analyse biochimique sont produites en conduisant une émission de chimioluminescence libérée à partir de la pluralité de régions absorbantes de l'unité d'analyse biochimique par une pluralité d'organes formant guides de lumière disposée de telle manière que chacune de la pluralité de régions absorbantes fasse face à l'une des portions terminales collectrices de lumière de la pluralité d'organes formant guides de lumière vers un détecteur de lumière et en détectant photoélectriquement l'émission de chimioluminescence par le détecteur de lumière.

5. Procédé de production de données d'analyse biochimique selon la revendication 4, dans lequel chacun de la pluralité d'organes formant guides de lumière est formé d'au moins une fibre optique.

6. Procédé de production de données d'analyse biochimique selon la revendication 4, dans lequel chacun de la pluralité d'organes formant guides de lumière est formé d'un faisceau de fibres optiques constitué par une pluralité de fibres optiques.

7. Procédé de production de données d'analyse biochimique selon l'une quelconque des revendications 4 à 6, dans lequel la pluralité d'organes formant guides de lumière est réunie au voisinage de portions terminales opposées aux portions terminales collectrices de lumière.

8. Procédé de production de données d'analyse biochimique selon l'une quelconque des revendications 4 à 7, dans lequel la pluralité d'organes formant guides de lumière est montée sur une tête de fixation au voisinage des portions terminales collectrices de lumière de sorte que chacune des portions terminales collectrices de lumière de la pluralité d'organes formant guides de lumière est disposée pour faire face à l'une de la pluralité de régions absorbantes.

9. Procédé de production de données d'analyse biochimique selon l'une quelconque des revendications 1 à 8, dans lequel le capteur de zone à semi-conducteur est constitué par un capteur de zone CCD refroidi.

10. Procédé de production de données d'analyse biochimique selon l'une quelconque des revendications 1 à 9, dans lequel l'unité d'analyse biochimique comprend un substrat formé d'une pluralité de trous devant être espacés les uns des autres et la pluralité de régions absorbantes est formée en chargeant un matériau absorbant dans la pluralité des trous formés dans le substrat.

11. Procédé de production de données d'analyse biochimique selon la revendication 10, dans lequel le substrat de l'unité d'analyse biochimique a la propriété d'atténuer l'énergie lumineuse.

**12.** Procédé de production de données d'analyse biochimique selon la revendication 11, dans lequel le substrat de l'unité d'analyse biochimique a la propriété de réduire l'énergie de la lumière à 1/5 ou moins lorsque la lumière parcourt le substrat sur une distance égale à celle entre les régions absorbantes voisines.

**13.** Procédé de production de données d'analyse biochimique selon la revendication 11 ou 12, dans lequel le substrat de l'unité d'analyse biochimique est constitué d'un matériau choisi dans le groupe consistant en un matériau métallique, un matériau céramique et un matériau plastique.

**14.** Procédé de production de données d'analyse biochimique selon l'une quelconque des revendications 1 à 13, dans lequel l'unité d'analyse biochimique est formée avec 10 ou plus régions absorbantes.

**15.** Procédé de production de données d'analyse biochimique selon l'une quelconque des revendications 1 à 14, dans lequel chacune de la pluralité de régions absorbantes est formée dans l'unité d'analyse biochimique pour avoir une taille inférieure à 5 mm$^2$.

**16.** Procédé de production de données d'analyse biochimique selon l'une quelconque des revendications 1 à 15, dans lequel la pluralité de régions absorbantes est formée dans l'unité d'analyse biochimique à une densité de 10 ou plus par cm$^2$.

**17.** Appareil de production de données d'analyse biochimique comprenant (i) un étage d'échantillon destiné à placer une unité d'analyse biochimique dans laquelle une pluralité de régions absorbantes sont formées de façon à être espacées les unes des autres et (ii) une substance dérivée d'un organisme vivant et marquée avec une substance marquante qui génère une émission de chimioluminescence lorsqu'elle vient en contact avec un substrat chimioluminescent est sélectivement liée avec des substances de liaison spécifiques dont la structure ou les caractéristiques sont connues et qui sont contenues dans la pluralité de régions absorbantes, (iii) un capteur de zone à semi-conducteur destiné à détecter photoélectriquement une émission de chimioluminescence libérée à partir de la pluralité de régions absorbantes de l'unité d'analyse biochimique et générer un signal analogique pour chacune de la pluralité de régions absorbantes de l'unité d'analyse biochimique, (iv) une pluralité d'organes formant guides de lumière disposés de telle manière que chacune des portions terminales collectrices de lumière des organes formant guides de lumière fasse face à chacune de la pluralité de régions absorbantes de l'unité d'analyse biochimique placée sur l'étage d'échantillon et adaptée pour conduire une émission de chimioluminescence libérée à partir de la pluralité de régions absorbantes de l'unité d'analyse biochimique vers le capteur de zone à semi-conducteur, (v) un convertisseur A/N destiné à numériser des signaux analogiques générés par le capteur de zone à semi-conducteur pour produire un signal numérique pour chacune de la pluralité de régions absorbantes de l'unité d'analyse biochimique, (vi) une mémoire destinée à stocker le signal numérique généré par le convertisseur A/N pour chacune de la pluralité de régions absorbantes de l'unité d'analyse biochimique, (vii) un moyen de traitement de données destiné à produire des données d'analyse biochimique pour chacune de la pluralité de régions absorbantes de l'unité d'analyse biochimique en se basant sur le signal numérique de chacune de la pluralité de régions absorbantes de l'unité d'analyse biochimique, et (viii) un moyen de commande destiné à commander le capteur de zone à semi-conducteur, le convertisseur A/N et le moyen de traitement de données, le moyen de commande étant adapté pour (a) commander le capteur de zone à semi-conducteur de façon à détecter photoélectriquement l'émission de chimioluminescence libérée à partir des régions absorbantes de l'unité d'analyse biochimique pendant une première période d'exposition, générant ainsi un signal analogique pour chacune des régions absorbantes de l'unité d'analyse biochimique et pour détecter photoélectriquement une émission de chimioluminescence libérée à partir des régions absorbantes de l'unité d'analyse biochimique pendant une seconde période d'exposition plus longue que la première période d'exposition, générant ainsi un signal analogique pour chacune des régions absorbantes de l'unité d'analyse biochimique, (b) commander le convertisseur A/N de façon à numériser le signal analogique de chacune des régions absorbantes de l'unité d'analyse biochimique généré par le capteur de zone à semi-conducteur et le stocker dans la mémoire, et (c) commander le moyen de traitement de données pour comparer une intensité de signal du signal numérique de chacune des régions absorbantes de l'unité d'analyse biochimique généré en détectant photoélectriquement une émission de chimioluminescence libérée à partir des régions absorbantes de l'unité d'analyse biochimique pendant la seconde période d'exposition et stocké dans la mémoire avec une valeur saturée de l'intensité de signal, pour déterminer l'intensité de signal du signal numérique qui est plus basse que la valeur saturée comme données d'analyse biochimique de la région absorbante de l'unité d'analyse biochimique, pour les stocker dans la mémoire, et (d) adopter une intensité de signal d'un signal numérique qui est généré en détectant photoélectriquement l'émission de chimioluminescence libérée à partir d'une région absorbante de l'unité d'analyse biochimique pendant la première période d'exposition et stockée dans la mémoire lorsqu'une intensité de signal d'un signal numérique généré en détectant photoélectriquement l'émission de chimioluminescence libérée à partir de la région absorbante de l'unité d'analyse biochimique pendant la seconde période d'exposition est égale ou supérieure à la valeur saturée, (e) produire ainsi des données d'analyse biochimique de la pluralité des régions absorbantes de l'unité d'analyse biochimique, **caractérisé en ce que** le moyen de traitement de données est constitué de façon à produire en outre un coefficient de correction (K) basé sur un rapport d'une intensité de signal (SC(2)) d'un signal numérique généré en détectant photoélectriquement une émission de chimioluminescence libérée à partir d'une région absorbante C de l'unité d'analyse biochimique pendant la seconde période d'exposition (T2) et stockée dans la mémoire et dont l'intensité de signal est plus basse que la valeur saturée et une intensité de signal (SC(1)) d'un signal numérique généré en détectant photoélectriquement une émission de chimioluminescence libérée à partir de la région absorbante C de l'unité d'analyse biochimique pendant la première période d'exposition T1 et stockée dans la mémoire, et pour multiplier une intensité de signal (SD(1)) du signal numérique généré en détectant photoélectriquement une émission de chimioluminescence libérée à partir d'une région absorbante D de l'unité d'analyse biochimique pendant la première période d'exposition (T1) et stockée dans la mémoire par le coefficient de correction (K) lorsque l'intensité de signal (SD(2)) du signal numérique généré en détectant photoélectriquement une émission de chimioluminescence libérée à partir de la région absorbante D pendant la seconde période d'exposition (T2) est égale ou supérieure à la valeur saturée produisant ainsi des données d'analyse biochimique de la région absorbante.

18. Appareil de production de données d'analyse biochimique selon la revendication 17, dans lequel le moyen de traitement de données est constitué de façon à diviser une intensité de signal d'un signal numérique dont l'intensité de signal est maximale parmi les intensités de signal de signaux numériques générés en détectant photoélectriquement une émission de chimioluminescence libérée à partir des régions absorbantes de l'unité d'analyse biochimique pendant la seconde période d'exposition et stockée dans la mémoire et dont l'intensité de signal est plus basse que la valeur saturée par une intensité de signal d'un signal numérique produit en détectant photoélectriquement une émission de chimioluminescence libérée à partir de la région absorbante de l'unité d'analyse biochimique pendant la première période d'exposition et stockée dans la mémoire, produisant ainsi le coefficient de correction.

19. Appareil de production de données d'analyse biochimique selon l'une quelconque des revendications 17 et 18, dans lequel chacun de la pluralité d'organes formant guides de lumière est formé d'au moins une fibre optique.

20. Appareil de production de données d'analyse biochimique selon l'une quelconque des revendications 17 et 18, dans lequel chacun de la pluralité d'organes formant guides de lumière est formé d'un faisceau de fibres optiques constitué par une pluralité de fibres optiques.

21. Appareil de production de données d'analyse biochimique selon l'une quelconque des revendications 17 à 20, dans lequel la pluralité d'organes formant guides de lumière est réunie au voisinage de portions terminales opposées aux portions terminales collectrices de lumière.

22. Appareil de production de données d'analyse biochimique selon l'une quelconque des revendications 17 à 21, dans lequel la pluralité d'organes formant guides de lumière est montée sur une tête de fixation au voisinage des portions terminales collectrices de lumière de sorte que chacune des portions terminales collectrices de lumière de la pluralité d'organes formant guides de lumière est disposée pour faire face à l'une de la pluralité de régions absorbantes.

23. Appareil de production de données d'analyse biochimique selon l'une quelconque des revendications 17 à 22, dans lequel le capteur de zone à semi-conducteur est constitué par un capteur de zone CCD refroidi.

# FIG.1

# FIG.2

EP 1 331 484 B1

# FIG.3

33

# FIG.4

# FIG.5

CCD **20**

A/D CONVERTER **21**

DATA BUFFER **22**

CAMERA CONTROL CIRCUIT **23**

COOLED CCD AREA SENSOR **15**

KEYBOARD **37**

CPU **30**

DATA TRANSFER MEANS — **31**

DATA PROCESSING MEANS — **32**

DATA STORING MEANS — **33**

DATA DISPLAY MEANS **34**

CRT **35**

EP 1 331 484 B1

# FIG.6

# FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1060784 A
- JP 1060782 A
- JP 4003952 A
- US 5422075 A